(19) Europäisches Patentamt

European Patent Office

Office européen des brevets

(11) **EP 2 540 818 A1**

(12) **EUROPEAN PATENT APPLICATION**

(43) Date of publication:
**02.01.2013 Bulletin 2013/01**

(21) Application number: **12183621.7**

(22) Date of filing: **04.09.2008**

(51) Int Cl.:
*C12N 1/20* (2006.01)   *A61K 35/74* (2006.01)
*A61P 11/00* (2006.01)   *A61P 11/06* (2006.01)
*A61P 37/08* (2006.01)   *A61P 13/00* (2006.01)
*A61P 1/00* (2006.01)   *A61P 31/04* (2006.01)
*C12R 1/25* (2006.01)   *C12N 1/06* (2006.01)
*C12R 1/225* (2006.01)

(84) Designated Contracting States:
**AT BE BG CH CY CZ DE DK EE ES FI FR GB GR
HR HU IE IS IT LI LT LU LV MC MT NL NO PL PT
RO SE SI SK TR**
Designated Extension States:
**BA MK**

(62) Document number(s) of the earlier application(s) in
accordance with Art. 76 EPC:
**08163693.8 / 2 161 329**

(71) Applicant: **OM Pharma
1217 Meyrin 2 (CH)**

(72) Inventors:
• **Bauer, Jacques Alain
1162 Saint-Prex (CH)**

• **Salvagni, Marco
1201 GENEVE (CH)**
• **Vigroux, Jean-Pierre Leon
74130 Bonneville (FR)**
• **Chalvet, Laetitia Leela Gisele
06130 St. Genis-Pouilly (FR)**
• **Chiavaroli, Carlo
01710 Thoiry (FR)**

(74) Representative: **Lecca, Patricia S.
Cabinet Lecca
21 rue de Fécamp
75012 Paris (FR)**

Remarks:
This application was filed on 08-09-2012 as a
divisional application to the application mentioned
under INID code 62.

(54) **Immunomodulatory extracts from lactobacillus bacteria and methods of manufacturing and use thereof**

(57) The present invention includes extracts from *Lactobacillus* bacteria, which may produce immunomodulatory effects in subjects. Embodiments of the invention may be used, for example, as nutraceuticals or pharmaceuticals for treatment of diseases or as adjuvants in medical treatment, such as those related to an imbalance of the production of anti-inflammatory or proinflammatory cytokines. Conditions for which extracts of the invention may be useful include infections, allergies, autoimmunity disorders, and inflammation, or as adjuvants providing healthful benefits in subjects. The invention also includes, *inter alia,* methods of making and using such extracts. The invention also relates to particular strains of *Lactobacillus* bacteria.

EP 2 540 818 A1

**Description**

Field of the Invention

Embodiments of the present invention include extracts from *Lactobacillus* bacteria, which may produce immunomodulatory effects in subjects. Embodiments of the invention may be used, for example, as nutraceuticals or pharmaceuticals for treatment of diseases, such as those related to an imbalance of the production of anti-inflammatory or proinflammatory cytokines, such as infections, allergies, autoimmunity disorders, and inflammation, or as adjuvants providing healthful benefits in subjects. The invention also includes, *inter alia,* methods of making and using such extracts. The invention also relates to particular strains of *Lactobacillus* bacteria.

**BACKGROUND AND SUMMARY OF THE INVENTION**

Immunomodulation is a global term that refers to a wide range of immune intervention that alters normal or abnormal immune responses. Microbes produce and secrete a wide range of molecules that can modulate eukaryotic immune responses (Lavelle et al., Curr Top Med Chem. 2004, 4(5), 499-508). These include factors that subvert protective mechanisms in order to facilitate pathogen colonization and persistence. Viral, bacterial and parasite-derived molecules that can inhibit inflammatory responses have been identified. In addition to microbial factors that may suppress immune responses, potent immune activators may also be of microbial origin themselves. These include bacterial enterotoxins, parasite-derived excretory-secretory products, and viral nucleic acids.

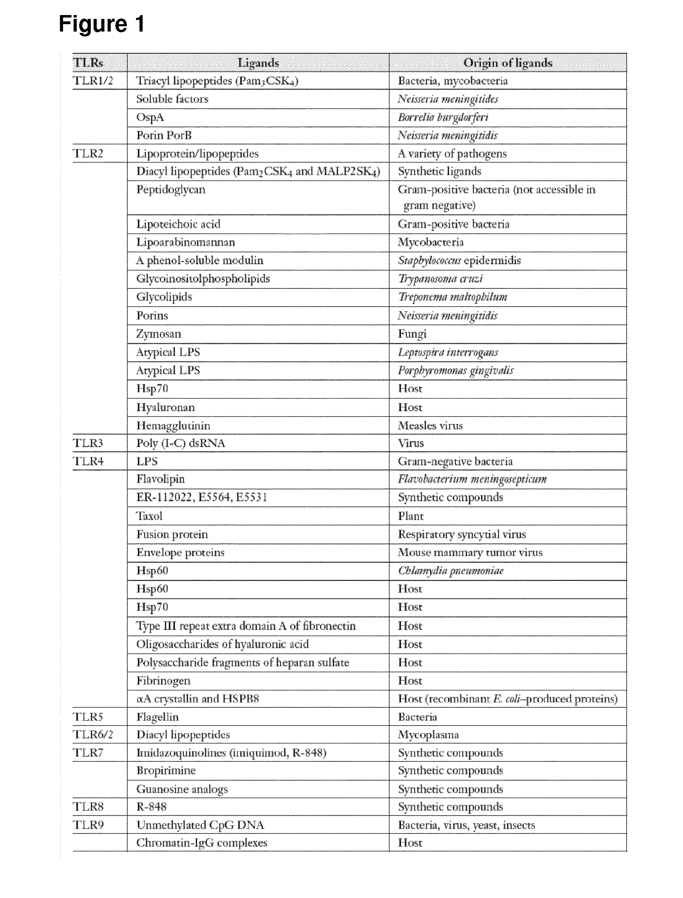

A family of at least 11 receptors, called toll-like receptors (TLRs) and expressed by the host organism, are thought to play a key role in immunological detection and innate responsiveness to microbes. Figure 1 provides a list of TLR ligands (Gay and Gangloff, Ann. Rev. Biochem., 2007, 76:141—65). TLRs recognize a wide range of molecules also known as pathogen-associated molecular patterns (PAMP) produced by viruses, bacteria and fungi (Tse and Horner, Ann Rheum Dis. 2007 Nov; 66 Suppl 3:iii77-80). TLR-linked immunomodulation has been applied in the development of novel therapies for a wide spectrum of pathologies, including infectious, malignant, autoimmune and allergic diseases.

TLR agonists and antagonists have been studied as potential therapeutics for the prevention and treatment of diseases. In relatively small clinical trials, TLR agonists have been used as adjuvants for vaccines aimed at preventing infections, extinguishing allergic hypersensitivities and clearing malignant cells. TLR agonists have also been investigated as monotherapies and adjunct therapies for the treatment of patients with infectious, allergic and malignant diseases. The use of TLR antagonists have also been studied in preclinical studies and clinical trials as potential therapeutics for autoimmune diseases and sepsis.

Probiotics are live microorganisms that may provide health benefits to a subject when administered in adequate amounts (Mottet et al., Digestive and Liver Disease, 2005, 37:3-6; Ezendam et al., Nutr Rev, Jan. 2006, 64(1):1-14; Gill and Prasad, Adv Exp Med Biol, 2008, 606:423-54). The biological mechanisms involved in immune response stimulation by probiotic microorganisms and certain cellular components of those microorganisms have been a subject of study. For example, gram-positive bacteria have a characteristic cell wall comprising macromolecules such as lipoteichoic acid (LTA). LTA may be associated with immunostimulatory activity (e.g., Bhakdi et al., Infect. Immun., 1991, 59:4614-4620; Setoyama et al., J Gen Microbiol, 1985, 131 (9):2501-2503; Cleveland et al., Infect Immun, 1996, 64(6):1906-1912). See also (Deininger et al., Clin Vaccine Immunol, 2007, 14(2):1629-1633). In addition, probiotic bacteria may contain a variety of TLR ligands with immunomodulatory characteriistics. Cell wall fragments from various bifidobacterial strains were found to stimulate interferon-gamma (IFN-γ) production *in vitro* in mouse splenocytes (T. Ambrouche, "Contribution à l'étude du pouvoir immunomodulateur des bifidobacteries: Analyse in vitro et étude ex vivo des mécanismes moléculaires impliqués," Ph.D. Thesis, Université Laval, Québec, 2005). Capsules made from particulatecellular wall fragments of particular lactic acid bacteria (Del-Immune V®, Pure Research Products, LLC, Colorado) are also intended to stimulate the immune system.

Injection of probiotic bacteria in live or killed form, or injection of particulate cell wall fragments of such bacteria, however, may not be the most efficacious way to provide an immunomodulatory effect in a subject, however. For instance, live cell extracts may comprise large proteins and lipopeptides whose size hinders efficient absorption by the subject, thus limiting the local concentration of helpful molecules from probiotic bacteria in the body. Conditions inside the body may also destroy active bacterial components or otherwise modify the chemical structures of those components, rendering them inactive. Risks associated with the oral administration of live probiotic microorganisms (*Lactobacillus*) include bacteremia and sepsis (Lactobacillus Sepsis Associated With Probiotic Therapy, Pediatrics, Jan. 2005, 115 (1):178-181). Hence, there is a need for other means to deliver the beneficial effects of probiotic bacteria to subjects in need thereof.

The present invention relates to *Lactobacillus* extracts, some embodiments of which may display strong immununomodulatory activities. For example, embodiments of the present invention relate to extracts from bacterial strains that may be useful as nutraceuticals or as pharmaceuticals, in some cases, to treat infectious diseases, allergy, respiratory disorders, and inflammatory pathologies, or to act as an adjunct in connection with a treatment protocol. The present invention also relates to compositions comprising the extracts, and processes of making the extracts, for example using media that do not pose a risk of prion diseases. Processes according to the invention include, for example, lysis of cells under alkaline conditions, or under alkaline conditions followed by acidic conditions. In some embodiments, the extracts of the invention are soluble extracts, meaning that they do not contain significant amounts of solid or particulate matter. In some embodiments, the extracts contain chemically modified TLR ligands. In some embodiments, alkaline treatment may cause chemical modification of cellular materials including TLR ligands, cell wall components, proteins, lipoteichoic acids, lipopeptides and phospholipids.

Some embodiments of the invention may comprise extracts obtained from one or more of the following species:

> *Lactobacillus fermentum, Lactobacillus rhamnosus, Lactobacillus plantarum,*
> *Lactobacillus johnsonii, Lactobacillus helveticus, Lactobacillus casei defensis,*
> *Lactobacillus casei ssp. casei, Lactobacillus paracasei, Lactobacillus bulgaricus,*
> *Lactobacillus paracasei, Lactobacillus acidophilus, Lactobacillus reuteri,*
> *Lactobacillus salivarius, Lactobacillus lactis,* and *Lactobacillus delbrueckii.*

In some embodiments, the extracts comprise at least one strain from each of the above species of bacteria, while in other embodiments, one or more specific strains from the list above may be removed or substituted with one or more different strains. Some embodiments of the present invention comprise an extract obtained from one or more of the following bacterial strains: *Lactobacillus fermentum 1-3929, Lactobacillus rhamnosus 71.38, Lactobacillus plantarum 71.39, Lactobacillus johnsonii 103782,* and *Lactobacillus helveticus 103146.* The strains above are deposited according to the Budapest Treaty. *Lactobacillus fermentum 1-3929, Lactobacillus rhamnosus 71.38, Lactobacillus plantarum 71.39, Lactobacillus johnsonii 103782,* and *Lactobacillus helveticus 103146* are each deposited at the Collection Nationale de Culture des Microorganismes at the Institut Pasteur, 25 rue du Dr. Roux, 75724 Paris, France. *Lactobacillus fermentum 1-3929* was deposited on February 27, 2008. The other strains are among the depository's collections and may be obtained by contacting the depository.

This invention also relates, *inter alia,* to the strain *Lactobacillus fermentum I-3929,* extracts obtained from that strain, methods of making such extracts, and the uses thereof. That strain was obtained by allowing strains from *Lactobacillus plantarum* and *Lactobacillus fermentum* to undergo chromosomal exchange, thus producing a novel *Lactobacillus* strain. Extracts obtained from *Lactobacillus fermentum* I-3929 were found to be active in several different *in vivo* and *in vitro* models correlating to infection and immunological disorders.

In some embodiments, an extract is obtained from only one specific bacterial strain. Alternatively, more than one strain may be used. In other embodiments, one or more extracts from a different type of microorganism, such as from an *non-Lactobacillus* bacterial species, may be added.

The extracts may be obtained by lysing bacterial cells in specific conditions after the cells are grown to a suitable concentration in a culture medium. In some embodiments, the bacteria are grown in a medium that does not pose a risk of prion-related diseases or a risk of other diseases that may be transmitted through ingesting products obtained from animal-based media. For example, in some embodiments a vegetable-based medium is used to grow the cells, such as a soya-based medium.

The lysates (i.e. the products of the cell lysis) may also be filtered to remove nucleic acids and larger cellular debris, such as insoluble or particulate matter. In some embodiments, the amount of nucleic acids present in the extracts is less than 100 $\mu$g/mL. Hence, in some embodiments, the resulting extract comprises soluble molecular components and does not contain significant amounts of insoluble or particulate material.

Membrane and cell wall molecules may be dissolved or suspended in the extracts, including lipoproteins, lipopeptides, peptidoglycans, lipooligosaccharides, lipoteichoic acids, and teichoic acids. During the lysis process, molecules in the cells, such as in membranes and cell walls, may become chemically modified, for example, cleaved into smaller structures, by alkaline treatment. In spite of such chemical modifications, embodiments of the invention may retain their biological activity in comparison to whole cells, or such embodiments may even demonstrate enhanced biological activities in comparison to whole cells.

For example, alkaline treatment may be used to lyse cells, or may be applied to cells that have previously been lysed by another method. During the alkaline treatment process according to some embodiments of the invention, L-amino acids found in natural proteins and lipopeptides are at least partially racemized to D-amino acids. D-amino acids can be beneficial in increasing the time of effectiveness of the extracts, as they are not efficiently digested in the mammalian gut. D-amino acids may also protect smaller peptides and proteins from degradation during digestion. Examples of D-amino acids include protein-bound D-amino acids, and to a lesser extent lysinoalanine (de Vrese et al., J Nutrition, 2000, 2026-2031). Thus, antigenic molecules in the extracts chemically modified during lysis to contain D-amino acids may remain in the patient's body for a longer time, potentially allowing for a stronger immunostimulating effect in some embodiments.

In some embodiments, a filtration process may also influence the properties of the resulting extracts, as the pore size of the filter, and in some cases, the chemical properties of the filter surface (i.e, its polarity), may alter the type of materials that are removed and retained. For example, some embodiments of the instant invention use a filtration process designed to retain molecules of interest but remove other molecular components such as nucleic acids or insoluble or particulate matter.

The filtered extracts may also be further purified by organic extraction, organo-aqueous extraction, chromatography, ultracentrifugation, ultrafiltration, or a combination of thereof.

## BRIEF DESCRIPTION OF THE DRAWINGS

Figure 1: Ligands for the family of 11 toll-like receptors (TLRs), expressed by the host organism.

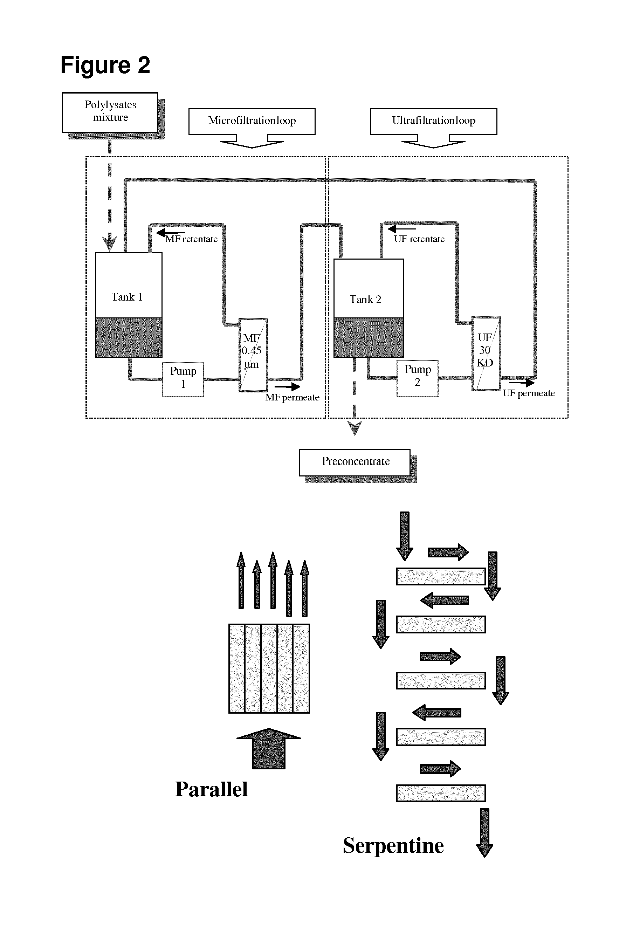

Figure 2: A diagram of a tangential flow filtration (TFF) system for preparation of bacterial extracts following lysis of bacteria. The diagram shows two different configurations for filters: a parallel mode where all filters work simultaneously and a serpentine mode where filters are configured in a serial mode.

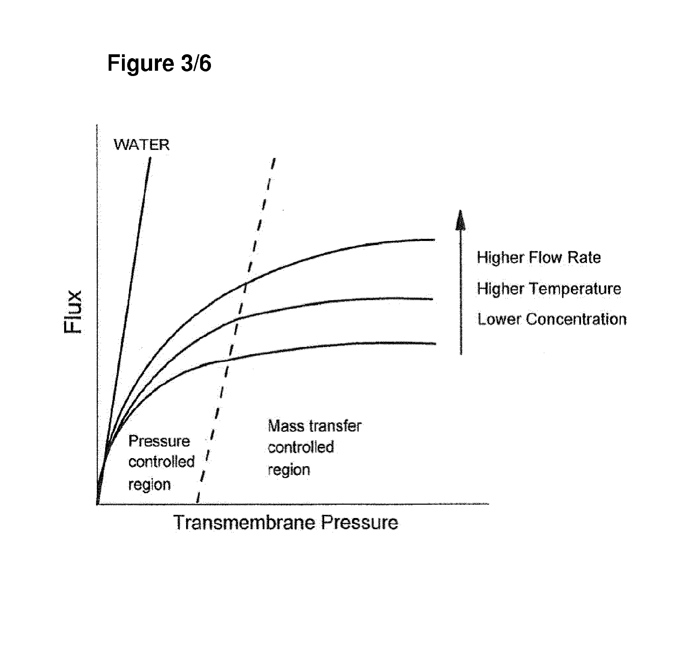

Figure 3: Generalized correlation between operating parameters and flux, inicating the areas of pressure control and mass transfer control for tangential flow filtration process (TFF).

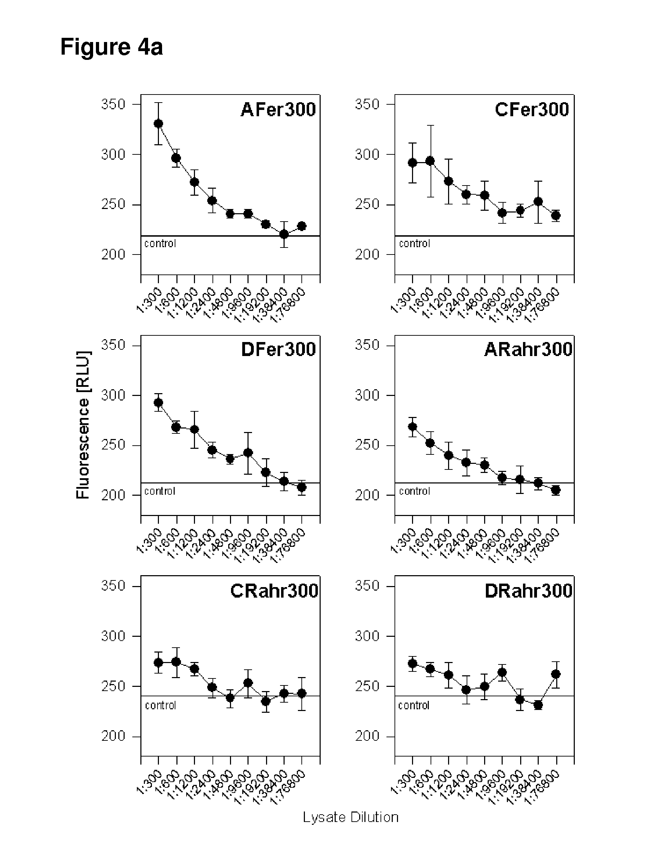

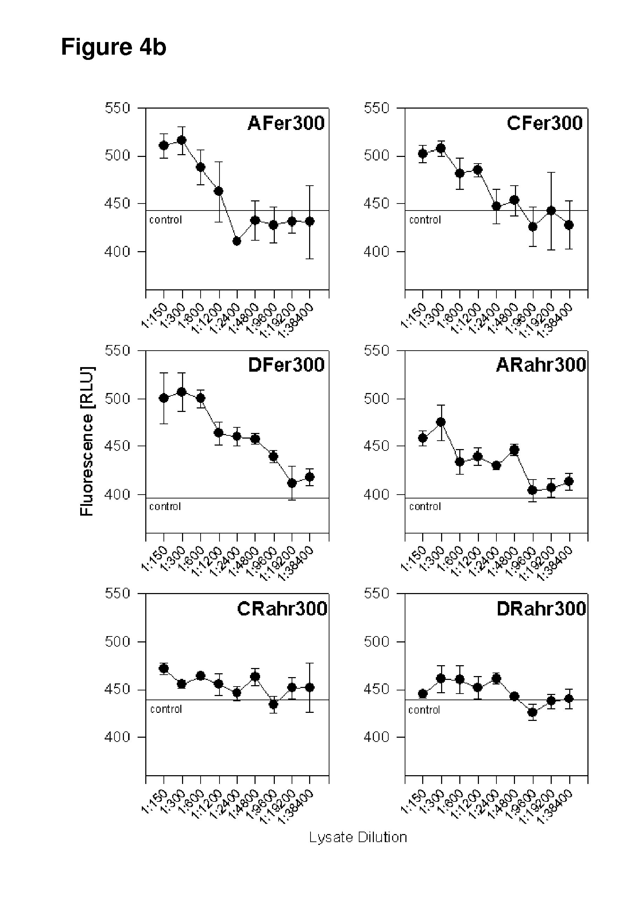

Figure 4: Stimulation of spleen cells cultured for 48 hours in the presence of different dilutions of the lysates AFer300, CFer300, and DFer300, and ARahr300, CRahr300, and DRahr300. After the addition of 30 $\mu$l/well Alamar blue® solution diluted 1:1 in cell culture medium, cells were further incubated (a) 8.5 h (first experiment); (b) 24 h (second experiment). Shown is the mean emission value at 590 nm $\pm$ standard deviation of duplicate cultures.

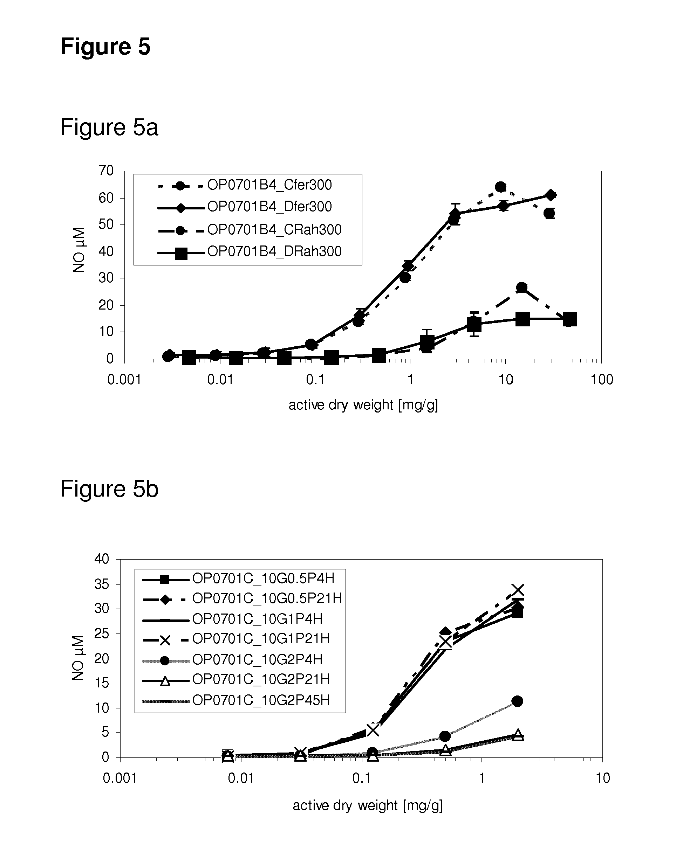

Figure 5: Induction of nitric oxide (NO) production in mice treated with extracts of *Lactobacillus fermentum* I-3929 and *Lactobacillus rhamnosus* 71.38 in (a) the first assay, and (b) the second assay. Results are expressed in $\mu$M nitric oxide (NO) as mean value $\pm$ standard deviation.

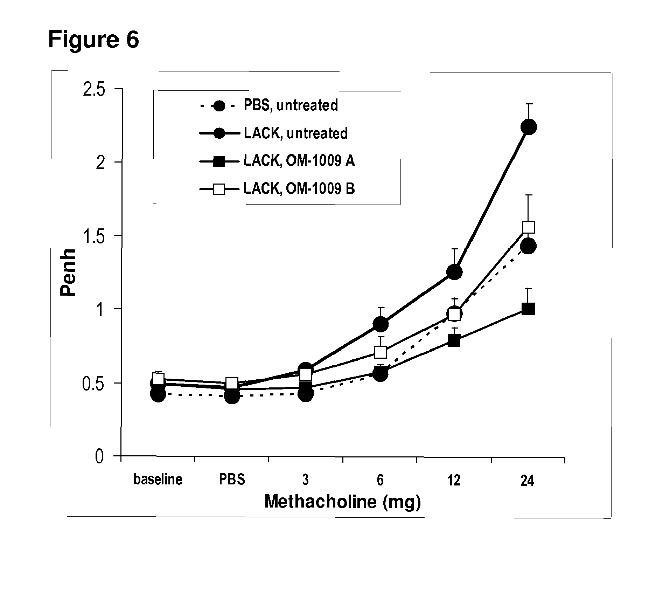

Figure 6: Effect of extracts of the invention on airway hyperresponsiveness (AHR) by whole-body plethysmography (Emka) with increasing concentrations of inhaled methacholine one day after the last antigen challenge. Results (in mean enhanced pause value +/- standard error of the mean) are shown for negative control animals treated with phosphate-buffered saline (PBS) (n=4), untreated LACK-challenged animals (as the positive control group, n=8), OM-1 009A-treated LACK challenged mice (n=8), and OM-1009B-treated LACK challenged mice (n=7).

## DETAILED DESCRIPTION OF THE INVENTION

Definitions

Extract: An extract, as defined herein, means material obtained following lysis of one or more bacterial strains. In some cases, the extract is obtained from only one strain while in others the extract is obtained from a mixture of several different strains.

In some cases, the extract is a soluble extract, meaning that it does not contain significant amounts of particulate and insoluble materials, such as particulate or solid cell wall fragments. Instead, components from cell walls, organelles, and cell membranes may be comprised in the extracts to the extent that they are dissolved or suspended. For example, the extract may be treated to remove particulate and insoluble materials, such as via filtration, centrifugation, or another separation technique.

Chemical lysis: This is a method of lysing bacterial cells under basic, acidic, and/or osmotic conditions.

Lysate: As used herein, this term means an extract of bacteria obtained from a cell lysis procedure.

Filtration: A filtration process, as described herein, means a passage of an extract or a mixture of extracts, through one or more filters such as microfilters (i.e., microfiltration) and/or ultrafilters (i.e., ultrafiltration). Such filtration may not necessarily remove 100% of the components it is designed to remove, but may, in some embodiments, render the extracts substantially free of those components. In some cases, filtration is repeated in several passes or cycles.

Initial pH: That term means the pH measured at the start of a procedure, such as bacterial lysis or filtration.

Saccharides: A saccharide, as defined herein, includes monosaccharides, disaccharides, as well as larger saccharides such as linear and branched polysaccharides. Saccharides also include substituted or chemically modified saccharides, such as lipopolysaccharides (LPS) and their chemically modified variants.

Lipoproteins: This term refers to macromolecules that comprise both protein or peptide chains and lipids, for example, a protein or peptide covalently bound to a lipid. Lipoprotein, as used herein, also includes lipopeptides.

Peptidoglycans: This term refers to polymers comprising sugars and amino acids.

Lipoteichoic acid (LTA): This term refers to a surface-associated adhesion amphiphilic molecule present in gram-positive bacterial strains.

Teichoic acid: This term refers to polymers of glycerol phosphate or ribitol phosphate linked together via phosphodiester bonds.

D-amino acids: This term refers to amino acids that exist in dextra-rotatory isomeric forms, as opposed to biosynthetically produced L-amino acids, which exist in levo-rotatory isomeric forms.

Racemization: This term indicates at least partial chemical modification of L-amino acids to D-amino acids.

Medium that avoids the risk of prion-based diseases means a culture medium used at any stage of the preparation of the extracts that does not comprise materials such as serum or meat extracts taken from animals such as cows or sheep, or from any other animal that can transmit prion-based diseases. Examples of such media include vegetable-based or synthetic media and also media using horse serum or media comprising materials taken from animal species that do not transmit prion diseases. Examples of prion-based diseases include, for example, mad cow disease, scrapie, and Creutzfeld-Jacob disease.

A non-animal medium is a medium that does not include components derived from animals. Examples include a vegetable-based (i.e. vegetal) medium, such as a soya medium, and a synthetic medium.

Nutraceutical, as used herein, means any composition that may have healthful effects in a subject upon administration wherein the composition is, for example, available to a subject without a doctor's prescription.

Treatment, as used in a therapeutic context herein, means both treatment of current diseases or disorders as well as prevention of or protection from the development of new diseases or disorders, for example.

Adjuvant, as used herein to refer to embodiments of the invention, refers to an embodiment of the invention provided to a subject in conjunction with a medical treatment plan.

Immunomodulation, immunomodulatory, and like terms, as used herein, refer to the ability to modify the immune responses in a subject in a manner that may have healthful benefits, such as to produce an anti-inflammatory or immunostimulatory effect.

Anti-inflammatory, and like terms, as used herein, refer to immunomodulatory effects serving to reduce inflammation.

Immunostimulatory and like terms, as used herein, refer to the stimulation of the immune system.

Protective immunity, as used herein, means that an embodiment is provided to a subject so as to provide protection from subsequent challenge with an infective agent or allergen. As a consequence, during challenge, the level of infective agent or allergen in the subject is sufficiently low in concentration so as to not significantly compromise the subject's health. The length of time in which such protection from challenge is effective may be limited, such as for a period of hours, days, or weeks.

Subject, as used herein, means any animal subject, including mammalian subjects, such as humans and domestic animals. Domestic animals, for example, may include mammals such as dogs, cats, horses, pigs, cows, sheep, goats, or other livestock, and also may include non-mammals such as birds, for example chickens, ducks, geese, turkeys and other livestock birds.

It is understood that the specific bacterial strains identified herein and used in the invention may include the strain obtained from the original deposit recited herein or a genetic clone thereof, including a strain that has been re-deposited at a later time with a different deposit code name, but which is considered to be genetically the same strain as the originally deposited version.

All numbers used herein are approximate, taking into account errors inherent in their measurement, rounding, and significant figures.

Preparation of Extracts

The present invention includes an extract of one or more *Lactobacillus* bacterial strains, wherein the extract is a soluble extract, and wherein the extract comprises chemically modified bacterial molecules.

The extracts of the present invention may be prepared, for example, by culturing of cells followed by harvesting the resulting biomass, lysis and purification. For each strain, to obtain a sufficient amount of material, the fermentation cultures may be started from a working seed lot followed by inoculation into larger fermentation containers.

The media used may be the same for each species. In some embodiments, a medium that avoids the risk of prion-based diseases may be used for growing all strains to be used.

After fermentation, the resulting biomass from one strain or from a set of strains may be inactivated by a heat treatment, concentrated, and frozen. Hence, the starting material used to form the extracts, in some embodiments, may be un-lysed whole cells.

In other embodiments, the starting material used to prepare the extracts may be biomass obtained from cells already at least partially lysed mechanically, enzymatically, or chemically. In yet other embodiments, the starting material may be a fraction of such previously lysed cells, such as a cell wall-containing fraction.

In some embodiments, the starting material is treated with an alkaline medium, such as from a strong base, such as hydroxide, or other strong mineral or organic bases. In this lysis or base treatment step, un-lysed cells in the starting material are lysed while, in some embodiments, cellular components may be chemically modified. Hence, in some embodiments, the chemically modified bacterial molecules are obtained by base treatment, such as strong base treatment of the one or more *Lactobacillus* bacterial strains from which the extract is obtained (i.e. base treatment of un-lysed cells or components or fractions from bacterial cells, as just explained).

In some embodiments, a biomass dry weight concentration of 2 to 90 g/L of may be subjected to base treatment, such as from about 2 to about 80 g/L, or from about 3 to about 40 g/L, such as 3, 5, 10, 15, 20, 25, 30, 35, or 40 g/L, or even about 5 to 50 g/L or other ranges bounded by the concentrations listed above. In some embodiments, about 40 to about 80 g/L is subjected to base treatment, such as 40, 50, 60, 70, or 80 g/L or ranges bounded by the concentrations listed above.

The biomass dry weight is defined here by the dry weight of material in g per liter of sample. It may be measured by drying the sample in a small porcelain dish at about 105 °C until it reaches a constant mass.

The temperature may be from 30 to 60°C, such as from 30 to 55°C, 30 to 50°C, 30 to 45°C, 30 to 40°C, or 30 to 35°C. In some embodiments, the base treatment temperature may be from 35 to 60°C, such as 35 to 55°C, 35 to 50°C, 35 to 45°C, or 35 to 40°C, for example. In some embodiments, the base treatment temperature may be 31°C, 32°C, 33 °C, 34 °C, 35 °C, 36 °C, 37°C, 38°C, 39°C, or even 40°C, or ranges bounded by the temperatures listed above.

The time of the base treatment may vary from 2 hours to several days, such as 1, 2, 3, 4, 5 or even 10 days, or from 3 to 120 hours, or from 3 to 48 hours, such as 3, 5, 8, 15, 14, 16, 18, 20, 22, 24, 26, 28, 30, 36, 40, 44, or 48 hours, or from 15 to 120 hours, such as 60 to 120 hours, such as 60, 72, 84, 96, 108, or 120 hours, or ranges bounded by the times listed above. It is understood that these ranges of time include any fractional number of days, hours, or minutes, therein.

In some embodiments, a strong base concentration of 0.001 N to 1.0 N is used, such as, from 0.001 N to 0.6 N, or from 0.10 N to 0.8 N, or from 0.6 N to 1.0 N, or a range starting or ending from 0.001, 0.002, 0.003, or 0.1 N, or from 0.1 N to 0.6 N, or a range starting or ending from 0.6, 0.7, 0.8, 0.9, 1.0, or 1.0 N, or other ranges bounded by the concentrations listed above. In some embodiments, a base concentration is used so as to achieve a initial pH of greater than 9.0, or a pH of greater than 9.5, a pH greater than 10.0 and less than 13.5, such as greater than 11.5, greater than 12.0, greater than 12.5, greater than 13.0, or from pH 9.0 to pH 13.5. In still other embodiments, a base concentration may be used so as to achieve an initial pH greater than 10.0 and less than 13.0, or from pH 9.0 to pH 13.0, for example.

In some embodiments, the pH during the base treatment may be decreased upon the extraction of soluble components. For example, the initial pH may be a basic pH, such as pH 9.0 to pH 13.0, or pH 9.5 to pH 12.5. The base treatment may be allowed to proceed for a certain period of time, such as 3 to 120 hours, such as 3 to 48 hours, or for a period of time as listed above, at a temperature as listed above. Then, in some embodiments, the pH may optionally be rendered acidic by the addition of, for instance, hydrochloric acid, so as to obtain a pH between 2.0 and 4.5, or a pH between 2.5 and 4.5, or a pH between 2.5 and 4.0, such as 2.5, 3.0, 3.5, 4.0, or a range bounded by any of the pH's listed above. The second treatment at low pH may be carried out at a temperature from 30 to 60°C, 35 to 55, or 35 to 45°C, such as 35°C, 36°C, 37°C, 38°C, 39°C, 40°C, 41°C, 42°C, 43°C, 44 °C, or even 45°C. The time of acidic treatment may vary from 1 hour to several hours up to 72 hours, for example between 1 hour and 24 hours, or between 1 hour and 6 hours, or between 3 hours and 48 hours, or between 3 hours and 24 hours, or between 4 and 72 hours, or even between 24 hours and 72 hours, or any range of time bounded by the times listed above.

In some embodiments of the invention, an alkaline treatment is performed on a bacterial biomass comprising, for example, material from *Lactobacillus fermentum,* having a biomass dry weight between 10 g /L and 40 g/L. In other embodiments, the alkaline treatment is performed on a bacterial biomass comprising a mixture of *Lactobacillus* strains and having a biomass dry weight between 10 g/L and 40 g/L. In such embodiments, the alkaline treatment may be performed at a hydroxide ion concentration between 0.025 N and 0.25 N, or at a pH between 9.5 and 12.5 at a temperature of 35 to 45°C for a time of between 3 hours and 48 hours. In some embodiments, the alkaline treatment is performed on bacterial biomass comprising material from one or more *Lactobacillus* strains at a hydroxoide ion concentration between 0.025 N and 0.20 N, between 0.025 and 0.15 N, between 0.025 and 0.10 N, between 0.05 N and 0.25 N, between 0.05 N and 0.20 N, between 0.05 and 0.15 N, between 0.05 N and 0.10 N, between 0.10 N and 0.25 N, between 0.10 N and 0.20 N, between 0.10 N and 0.15 N, between 0.15 N and 0.25 N, between 0.15 N and 0.20 N, or even between 0.20 N and 0.25 N. Such embodiments may, for example, have a pH between 9.5 and 12.0, between 9.5 and 11.5, between 9.5 and 11.0, between 9.5 and 10.5, between 9.5 and 10.0, between 10.0 and 12.5, between 10.0 and 12.0, between 10.0 and 11.5, between 10.0 and 11.0, between 10.0 and 10.5, between 10.5 and 12.5, between 10.5 and 12.0, between 10.5 and 11.5, between 10.5 and 11.0, between 11.0 and 12.5, between 11.0 and 12.0, between 11.0 and 11.5, between 11.5 and 12.5, between 11.5 and 12.0, or even a pH between 12.0 and 12.5. The time of alkaline treatment for such embodiments may be between 3 hours and 36 hours, between between 3 hours and 24 hours, between 3 hours and 18 hours, between 3 hours and 12 hours, between 3 hours and 6 hours, between 6 hours and 48 hours, between 6 hours and 36 hours, between 6 hours and 24 hours, between 6 hours and 18 hours, between 6 hours and 12 hours, between 6 hours and 8 hours, between 8 hours and 48 hours, between 8 hours and 36 hours, between 8 hours and 24 hours, between 8 hours and 18 hours, between 8 hours and 12 hours, between 12 hours and 48 hours, between 12 hours and 36 hours, between 12 hours and 18 hours, between 18 hours and 48 hours, between 18 hours and 36 hours, between 18 hours and 24 hours, between 24 hours and 48 hours, between 24 hours and 36 hours, or between 36 hours and 48 hours. The alkaline treatment may be performed for any of the time periods bounding the ranges above, for example, 3, 6, 8, 12, 18, 24, 36, or even 48 hours. Such conditions may provide for a moderate alkaline treatment.

In other embodiments, 10 g/L and 40 g/L of biomass dry weight from one or more *Lactobacillus* strains may be subjected to a hydroxide ion concentration between 0.15 N and 0.50 N or a pH between 11.5 and 13.5, at a temperature of 35 to 45°C for a time of between 15 hours and 120 hours. For example, in some embodiments, the hydroxide concentration may be between 0.15 N and 0.45 N, between 0.15 N and 0.40 N, between 0.15 N and 0.35 N, between 0.15 N and 0.30 N, between 0.15 N and 0.25 N, between 0.15 N and 0.20 N, between 0.20 N and 0.50 N, between 0.20 N and 0.40 N, between 0.20 N and 0.30 N, between 0.25 N and 0.50 N, between 0.30 N and 0.50 N, between 0.30 N and 0.40 N, or between 0.40 N and 0.50. Such embodiment may have a pH between 11.5 and 13.0, between 11.5 and 12.5, between 11.5 and 12.0, between 12.0 and 13.5, between 12.0 and 13.0, between 12.0 and 12.5, between 12.5 and 13.5, between 12.5 and 13.0, between 13.0 and 13.5, for example. The time period for alkaline treatment may be between 15 hours and 100 hours, between 15 hours and 90 hours, between 15 hours and 75 hours, between 15 hours and 60 hours, between 15 hours and 48 hours, between 15 hours and 36 hours, between 24 hours and 120 hours, between 24 hours and 100 hours, between 24 hours and 90 hours, between 24 hours and 75 hours, between 24 hours and 60 hours, between 24 hours and 48 hours, between 36 hours and 120 hours, between 36 hours and 100 hours, between 36 hours and 90 hours, between 36 hours and 75 hours, between 36 hours and 60 hours, between 36 hours and 48 hours, between 48 hours and 120 hours, between 48 hours and 100 hours, between 48 hours and 90 hours, between 48 hours and 75 hours, between 48 hours and 60 hours, between 60 hours and 120 hours, between 60 hours and 100 hours, between 60 hours and 90 hours, between 60 hours and 75 hours, between 75 hours and 120 hours, between 75 hours and 100 hours, between 75 hours and 90 hours, between 90 hours and 120 hours, or between 100 and 120 hours, for example. Time periods also contemplated for alkaline treatment in such embodiments include 15, 24, 48, 60, 75 90, 100, and 120 hours. Such conditions may provide for a strong alkaline treatment.

In other embodiments, between 10 g/L and 40 g/L of starting biomass dry weight may be treated with a hydroxide concentration between 0.025 N and 0.25 N or a pH between 9.5 and 12.5, at a temperature of 35 to 45°C for a time of between 3 hours and 48 hours. The pH may then be adjusted to between 2.5 and 4.0 by the addition of acid, such as hydrochloric acid (HCl), comprising an acidic treatment. The acidic treatment may be performed at a temperature of between 35 and 45°C for a time of between 1 hour and 24 hours. For example, in such embodiments, the alkaline treatment of bacterial biomass comprising one or more *Lactobacillus* strains may be performed with a hydroxide concentration between 0.025 N and 0.20 N, between 0.025 and 0.15 N, between 0.025 and 0.10 N, between 0.05 N and 0.25 N, between 0.05 N and 0.20 N, between 0.05 and 0.15 N, between 0.05 N and 0.10 N, between 0.10 N and 0.25 N, between 0.10 N and 0.20 N, between 0.10 N and 0.15 N, between 0.15 N and 0.25 N, between 0.15 N and 0.20 N, or even between 0.20 N and 0.25 N. During the alkaline treatment, such embodiments may, for example, have a pH between 9.5 and 12.0, between 9.5 and 11.5, between 9.5 and 11.0, between 9.5 and 10.5, between 9.5 and 10.0, between 10.0 and 12.5, between 10.0 and 12.0, between 10.0 and 11.5, between 10.0 and 11.0, between 10.0 and 10.5, between 10.5 and 12.5, between 10.5 and 12.0, between 10.5 and 11.5, between 10.5 and 11.0, between 11.0 and 12.5, between 11.0 and 12.0, between 11.0 and 11.5, between 11.5 and 12.5, between 11.5 and 12.0, or even a pH between 12.0 and 12.5. The time of alkaline treatment for such embodiments may be between 3 hours and 36 hours, between between 3 hours and 24 hours, between 3 hours and 18 hours, between 3 hours and 12 hours, between 3 hours and 6 hours, between 6 hours and 48 hours, between 6 hours and 36 hours, between 6 hours and 24 hours, between 6 hours and 18 hours, between 6 hours and 12 hours, between 6 hours and 8 hours, between 8 hours and 48 hours, between 8 hours and 36 hours, between 8 hours and 24 hours, between 8 hours and 18 hours, between 8 hours and 12 hours, between 12 hours and 48 hours, between 12 hours and 36 hours, between 12 hours and 18 hours, between 18 hours and 48 hours, between 18 hours and 36 hours, between 18 hours and 24 hours, between 24 hours and 48 hours, between 24 hours and 36 hours, or between 36 hours and 48 hours. The alkaline treatment may be performed for any of the time periods bounding the ranges above, for example, 3, 6, 8, 12, 18, 24, 36, or even 48 hours. The pH may then be adjusted to between 2.5 and 3.5, between 2.5 and 3.0, between 3.0 and 4.0, between 3.0 and 3.5, or between 3.5 and 4.0 by the addition of acid, for the acidic treatment following the alkaline lysis. The acidic treatment may be performed for a time of between 1 hour and 18 hours, between 1 hour and 12 hours, between 1 hour and 6 hours, between 1 hour and 3 hours, between 3 hours and 24 hours, between 3 hours and 18 hours, between 3 hours and 12 hours, between 3 hours and 6 hours, between 6 hours and 24 hours, between 6 hours and 18 hours, between 6 hours and 12 hours, between 12 hours and 24 hours, between 12 hours and 18 hours, between 18 hours and 24 hours. Times also contemplated for the acidic treatment include 1, 3, 6, 12, 18, and 24 hours.

The lysates obtained following the base treatment described above may next be purified by centrifugation and/or filtration, for example, to remove particulate and insoluble components. For example, lysates may be centrifuged at 9000 x gravity, followed by one or more rounds of filtration on a 0.2 micron filter. In some cases, successive rounds of filtration on larger pore filters followed by filtration on a 0.2 micron filter may be used. Ultrafiltration methods may also be employed in order to help extract soluble materials from the extract, for example, recirculating the ultrafiltration permeate for further microfiltration.

In some embodiments, a tangential flow filtration (TFF) method may be used to filter the lysates and to extract soluble molecules from larger cellular debris (Figure 2). See, e.g., Separations Technology, Pharmaceutical and Biotechnology Applications, Wayne P. Olson, Editor. Interpharm Press, Inc., Buffalo Grove, IL, U.S.A., p.126 to 135 — ISBN:0-935184-72-4. At the beginning of such a process, a diluted bacterial lysate may be stored in a first tank. In TFF, for example, the extract may be exposed to both a microfilter and an ultrafilter. For example, a microfiltration (MF) loop is started, and the product is pumped. The resulting MF retentate is recycled, while the MF permeate is transferred to a second tank.

After reaching a suitable degree of concentration, an ultrafiltration (UF) loop is started. The UF permeate may be recirculated back to the first tank for continuous extraction of solubilized extracts from the lysate while the UF retentate is stored in the second tank. During the continuous extraction, the volumes in tanks 1 and 2 may be adjusted by regulation of flow rates of the microfiltration and ultrafiltration permeates.

Several such extraction cycles may be performed, either with TFF or another filtration method. In embodiments that use TFF, at the end of the last cycle, the ultrafiltration loop may be shut down and the microfiltration loop may be run alone and the MF permeate transferred to tank 2.

Conditions of crossflow and transmembrane pressure are defined by the pressure independent and mass transfer controlled regions in film theory described, for example, by M.Cheryan (Ultrafiltration and Microfiltration Handbook, 2nd Ed., Ch. 4, 1998). The permeate flux and extraction yield are affected by filtration conditions (transmembrane pressure (TMP), crossflow, temperature, etc.). The type of filter can also affect the filtration performance, as well as the type of plate system (cassette filter). Different configurations, including parallel mode and serpentine mode can be used (see Figure 2). Specific conditions are developed for optimized performances for each combination of type of mode and type of filter used.

The microfiltration loop may be fitted with filters of 1.2 microns to 0.1 microns, such as filters of 0.65 to 0.2 microns, or 0.45 microns. The cross-flow may be between 100 and 3000 Liters/ hours $m^2$ (LHM), such as between 300 and 2500 LHM, or 2000 LHM with a TMP of 0.3 to 2 bar. The ultrafiltration loop may be fitted with filters of from 10 KDa to 1000 KDa, such as from 10 KDa to 100 KDa, or from 10 KDa to 30 KDa, or from 30 KDa to 100 KDa. The cross-flow may be between 30 and 1000 LHM, such as between 20 and 500 LHM with a TMP of 0.2 to 1.5 bar.

Between 5 and 20 diafiltration volumes may be used to extract soluble components from bacterial cell walls. In some embodiments, between 8 and 15 volumes are used. Hence, for example, in some embodiments, between 5 and 15 cycles of filtration may be used, in some cases between 8 and 15 cycles.

Following filtration, the extract may be further concentrated or centrifuged, if desired. For instance, a further microfiltration using a smaller pore filter may be performed, such as a 0.2 micron filter. Following filtration, the extract may be lyophilized prior to formulating it for use.

In some embodiments, following the filtration, the extract can be purified in order to separate, to eliminate or increase the concentration of one or more modified components in the extract. For instance, a strong ionic chromatography step can be used in order to remove charged components. Other purification processes can be used, such as gel filtration, chromatography, ultracentrifugation, extraction and precipitation.

Chemical Properties of Bacterial Extracts

Base treatment may result in a variety of chemical modifications to cellular components. For example, in proteins: (1) peptide bonds may undergo partial cleavage generating smaller polypeptides; (2) natural L-amino acids may be at least partially racemized to D-amino acids; and (3) asparagine and glutamine residues may become deaminated, leading to changes in the protein isoelectric point. Molecules such as lipoteichoic acids, lipopeptides, and phospholipids may undergo a base-catalyzed hydrolysis of ester bonds and/or amide bonds leading to modified amphiphilic structures which may have new physicochemical and immunological properties. Examples of other possible chemical modifications include partial solubilization of cell wall polysaccharides and complete hydrolysis of ribonucleic acid (RNA) into individual ribonucleotides, including rearrangement of phosphate groups.

Hence, some or all of those chemical modifications may occur during base treatment of *Lactobacillus* cells as described herein. Such molecular modifications may affect the biological activities of the extracts.

For example, base treatment of bacteria according to the present invention may result in partial hydrolysis of proteins as well as deamination, deamidation, and/or partial racemization of amino acids from L to D. In one analytical study of an extract according to the invention, peaks representing D-aspartic acid, D-glutamic acid, D-serine, D-methionine, D-histidine, D-alanine, D-arginine, D-phenylalanine, D-tyrosine, D-leucine, and D-lysine, were each observed. The percentage of D-amino acids of those species in that study ranged from 3% to 40%. Hence, some embodiments of the invention allow for racemization of one or more of serine, threonine, histidine, alanine, arginine, tyrosine, phenylalanine, leucine, and lysine, such as all of the above amino acids, or any selection of more than one but less than all of the above amino acids, such as, for example, alanine, phenylalanine and lysine. In some embodiments, at least 10% of one or more of the above amino acids may become racemized from D to L. In other embodiments, at least 40% of one or more of the above amino acids may become racemized.

Thus, extracts of the present invention may comprise between 1 and 90% D-amino acids, such as between 1 and 80%, or between 1 and 60%. In some embodiments, the extract comprises between 10 and 45% D-amino acids, such as between 25 and 35% D-amino acids. The extracts of the invention may comprise at least one D-amino acid selected from the group consisting of D-aspartic acid, D-asparagine, D-glutamic acid, D-glutamine, D-serine, D-methionine, D-histidine, D-alanine, D-arginine, D-phenylalanine, D-tyrosine, D-leucine, D-lysine, D-valine, and D-threonine. In some embodiments, the concentration of any one D-amino acid comprises between 1 and 50 %, such as between 10 and 40 %, or even between 15 and 35 %.

Some extracts of the present invention comprise *Lactobacillus* bacterial cell wall and membrane components, such as lipoteichoic acids, teichoic acid, peptidoglycan, or a combination thereof. In some embodiments, those components are chemically modified. Some extracts also comprise cell wall and/or cell membrane components such as lipoproteins, which may also be chemically modified. In some embodiments, the cell wall components or cell membrane components, for example lipoproteins, are dissolved or suspended in the extracts and thus are not present in particulate or insoluble form.

In addition, an extract according to the present invention may comprise, for example, from 10 to 100 mg/mL soluble dry weight (SDW) of material, 1 to 30 mg/mL of protein (Prot.), 0.5 to 4.0 mg/mL of sugar and less then 100 $\mu$g/mL DNA. For example, some embodiments contain about 15 to 35 mg/mL of soluble dry weight, 3 to 7 mg/mL of protein, 1.0 to 3.0 mg/mL of sugar and 10 to 40 $\mu$g/mL of DNA. An extract according to the present invention may contain, for example, 30 mg/mL of soluble dry weight, 9.6 mg/mL protein, 2.4 mg/mL of sugar and 33 $\mu$g/mL of DNA or another example containing 32.4 mg/mL of soluble dry weight, 5.8 mg/mL protein, 2.3 mg/mL of sugar and less than 100$\mu$g/mL of DNA. Soluble Dry Weight (SDW) in g/L or mg/mL is determined by obtaining 5 mL of the soluble fraction resulting from the lysis or base treatment and drying it to a constant mass in a porcelain dish at 105°C.

In some embodiments, the extracts comprise at least 0.3 mg/mL of saccharides, such as between 0.3 and 4.5 mg/mL of saccharides. In some embodiments, at least one saccharide is chosen from monosaccharides, disaccharides, and polysaccharides. Some extracts of the invention comprise at least one branched polysaccharide. In some embodiments, at least one saccharide is chemically modified.

Lysis or base treatment of bacteria according to the present invention may result in a decrease of the average molecular weight of component macromolecules to a range of, for instance, 1 kDa to between 300 kDa and 100 kDa, or to a range of 1 kDa to between 60 kDa and 10 kDa. In some embodiments, the extract comprises at least one protein with a molecular weight of less than 50 kDa or less than 30 kDa, such as less than 10 kDa.

Biological Activities of Bacterial Extracts

Extracts according to the invention may have immunomodulatory activities. For example, some extracts may stimulate the immune system. Some extracts may have anti-inflammatory activities. The specific effects of an extract may depend upon the manufacturing conditions and the species or strain of *Lactobacillus,* or mixture of species or strains, from which the extract is obtained. Accordingly, some extracts according to the invention may show potent immunostimulatory activity, and may thus be useful in treating infections, or as adjuncts to such treatment, while other embodiments may show weaker immunostimulatory activity but show anti-inflammatory activity, thus being useful in treating inflammatory disorders such as allergies, asthma, autoimmune diseases, colitis, and inflammatory bowel diseases, or as adjuncts to such treatment.

Thus, some extracts according to the invention may be effective to treat patients suffering from disorders including, but not limited to, microbial infections, allergic diseases, and digestive tract disorders. Some extracts according to the invention may also be provided to a patient as nutraceuticals, for example, as adjuvants in the treatment of a variety of conditions including, but not limited to, microbial infections, allergic diseases, and digestive tract disorders.

The range of the biological activiies of the extracts may be determined by several *in vitro* and *in vivo* assays. For example, the AlamarBlueTM-Assay incorporates a fluorometric/colorimetric growth indicator based on the detection of metabolic activity by an oxidation-reduction (REDOX) indicator in response to chemical reduction resulting from cell growth (Example 4).

*In vitro* cell assays test the production of nitric oxide (NO) from primary murine macrophages and can screen for the ability of an extract to stimulate the immune system in order to kill invading bacteria (Figure 5). In some embodiments, the extracts may stimulate NO production in murine macrophages, leading to measured NO concentrations ranging from 3 $\mu$M to 60 $\mu$M, such as 5 $\mu$M to 40 $\mu$M. In some embodiments, the NO concentration may be above 30 $\mu$M. The type of bacterial species may also affect these results. For example, extracts from *Lactobacillus fermentum* may induce a larger nitric oxide production than *Lactobacillus rahmnosus* (see, e.g. Example 5 below). In order to screen embodiments of the invention for their immunostimulating or anti-inflammatorypotential *in vitro,* tests of the bacterial extracts of the invention may be performed on human peripheral blood mononuclear cells (PBMC). See Foligne et al. (World J Gastroenterol, 2007, 13(2):236-243). The release of both IL12p70 (an inflammatory cytokine) and IL10 (an anti-inflammatory cytokine) may be measured, and the IL-10/IL-12 ratio calculated (Example 6). Some embodiments of the invention produce a higher IL10/IL12 ratio than a live *Lactobacteria fermentum* control, thus suggesting that some extracts of the invention may display an equivalent, or even stronger anti-inflammatory properties than the live parent microorganism when administered *in vivo.* Hence, the invention includes extracts capable of achieving a calculable IL10/IL12 ratio in human peripheral blood mononuclear cells, wherein the ratio is equal to or greater than the IL10/IL12 ratio achieved by a live *Lactobacillus* strain from which the extract is obtained.

The immune responses of the extracts of the invention may also be tested by examining their effects on Toll-like receptors (TLRs), For example, extracts may be tested in HEK293 cells in the presence or absence of the TLR2 agonist Pam3Cys, or in the presence or absence of the TLR4 agonist LPS (Example 7). The HEK293 cell line enables efficient monitoring of TLR activity using ELISA analyses such as IL-8 titration or reporter-based systems that monitor TLR-induced NF-κB activation. Some embodiments of the invention may act as TLR 2/6 antagonists in HEK TLR 2/6 cells. Thus some embodiments may be useful to fight infections in a subject, whereas other embodiments may be developed for use against inflammation and/or autoimmunity disorders.

The extracts presently disclosed may also be screened for TLR and NOD2 receptor activity (Example 8). Some embodiments of the invention activate TLR and or NOD2 receptors *in vitro,* indicating that they may be able to activate the immune system, via TLRs and/or NOD2.

The plaque forming cells (PFC) technique may be employed to evaluate a non-specific stimulation of B-lymphocytes (Example 9). Certain lymphoid cells release hemolytic antibodies which diffuse and cause the lysis of the neighboring red blood cells by forming a lysis plaque in the presence of complement. Some embodiments of the invention may increase the secretion of immunoglogulins by B cells, and thus can potentially be used prophylactically for priming the immune system in subjects suffering from recurrent infections.

The anti-infective efficacy of the extracts of the instant invention may be tested, for example upon *Salmonella* infection of subjects, such infection of mice (Example 10). Some embodiments of the invention may provide protective immunity against infections, such as bacterial infections, i.e. *Salmonella* infections. For example, some embodiments may decrease the mortality induced in mice by the injection of *Salmonella thyphimurium.*

Combination of the NO *in vitro* activity with determination of an *in vivo* activity measured, e.g., in a murine model of allergen-insuced asthma (Example 11) may provide a more complete view of the potential clinical activity of the extracts presently disclosed. In the LACK (protein from the parasite *Leishmania* major) model, the number of eosinophils found in broncho-alveolar lavage fluids when compared to asthmatic control (non-treated) animals may be decreased by a factor between 1 and 10, such as decreased 1.5-fold to 5-fold. Accordingly, in some embodiments, the extract decreases the eosinophil cell number, neutrophil cell number, lymphocyte cell number, or any combination thereof, in an asthmatic murine subject by a factor of at least 1.5 with respect to an asthmatic non-treated control. Some embodiments of the invention may decrease eosinophilia in asthmatic subjects, and concomitantly decrease the level of Th2 cytokines (such as IL4, IL5, IL13), which are markers of asthma. Thus, such embodiments, for example, may have anti-inflammatory activities in subjects suffering from immunological disorders, such as allergic disorders, including asthma.

Compositions Comprising the Bacterial Extracts

Extracts according to the invention may be formulated in a number of different ways for eventual administration. For example, oral tablets, capsules, pills, may be prepared, as well as liquid formulations or aerosols. Formulations for infusion or injection may also be prepared.

Embodiments of this invention can be formulated, for example, as solid dosage forms or liquid dosage forms. Exemplary solid dosage forms may include, for example, a tablet, e.g. coated tablet, chewable tablet, effervescent tablet, sublingual tablet, granulates, powder, or a capsule) containing the extract.

Solid dosage forms may also contain diluents, fillers, and/or other excipients. Other excipient components may be added such as preservatives, colorants, flavourings, and sweeteners.

As an alternative to the capsules and the tablets, it is possible to develop powder or granulates formulations. Liquid dosage forms as solution or syrup, suspension and drops can also be developed for the oral route.

The extracts of the present invention may be included in one or more nutraceutical compositions, such as nutritional and/or dietary supplements and food additives, or one or more pharmaceutical compositions.

Administration of the Bacterial Extracts to a Subject

A dose comprising at least one extract of the present invention may be administered to a subject suffering from or at risk of developing at least one disorder chosen from digestive tract disorder, respiratory tract disorder, urinary tract disorder, and allergic conditions. For example, in some embodiments, the extracts may be administered to subjects suffering from, or at risk of developing, pulmonary upper an lower infections, obstructive pulmonary disease with acute lower respiratory infection, obstructive pulmonary disease with acute exacerbation, nasopharyngitis, sinusitis, pharyngitis, tonsillitis, laryngitis, tracheitis, laryngopharyngitis, influenza, pneumonia, bronchopneumonia, bronchitis, rhinitis, nasopharyngitis, pharyngitis, sinusitis, tonsillitis, laryngitis, laryngotracheitis, bronchitis, allergic rhinitis, allergic asthma, atopic dermatitis, urinary tract infections due to obstructive and reflux uropathy, urethritis, tubulo-interstitial nephritis, obstructive pyelonephritis, cystitis including chronic cystitis, male pelvic pain syndrome including prostatitis and chronic prostatitis, prostatocystitis, female pelvic inflammatory diseases, Crohn's disease, and/or irritable bowel syndrome.

In some embodiments, the extracts are administered to a subject in the form of a nutraceutical composition such as a nutritional supplement and/or food additive. In other embodiments, the extracts are administered to a subject in the form of a pharmaceutical composition. The administration may comprise a single dose or multiple dose administration.

In some embodiments, an extract may be provided in a therapeutically effective dose to treat a subject suffering from one or more of the conditions above. In some embodiments, an extract may be provided as an adjunct to other medical treatment.

## WORKING EXAMPLES

### Example 1: Bacterial Cultures

Example 1.1: Culture of *Lactobacillus fermentum* I-3929

Initial Culture Conditions

Culture media was prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid: 1.1 mg/L): 0.5 mL/L. Polypropylene glycol (0.02 mL/L, density 1.005 g/mL) was then added. After dissolution, the pH was not adjusted. After sterilizing the media, small Erlenmeyer flasks were individually inoculated with the content of frozen vials (containing 1.5 mL of frozen bacteria) and incubated at 37°C for 8 hours. Then 20 ml aliquots of this culture were transferred to larger Erlenmeyer flasks containing 1000 mL of culture media, and incubated again in the same conditions. After 16 hours growth, the content of the 1 liter Erlenmeyer flask was transferred to the prefermenter.

Culture Conditions in Prefermenters

20 liters of culture media were prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid: 1.1 mg/L): 0.5 mL/L. Polypropylene glycol (0.02 mL/L) was then added. After dissolution, the pH was not adjusted. The incubation temperature was regulated at 37°C, with 100 rpm stirring and no aeration. The pH was not regulated during the culture. After 24 hours, 7 liters from the prefermenters were transferred to a fermenter (optical density (OD) at 700 nm prefermenter culture after 24 hours = 1.24). The cultures of the prefermenters were transferred under sterile conditions into fermenters.

Culture Conditions in Fermenters

70 liters of culture media were prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate : 0.2 g/L; Metal solution (copper sulfate: 3 mg/l; iron chloride: 830 mg/l; zinc sulfate: 860 mg/l; sulfuric acid : 1.1 mg/L): 0.5 mL/L. Polypropylene glycol (0.02 mL/L) was then added. After dissolution, the pH was not adjusted.

After sterilization, 8 g/L glucose was added to the culture. The incubation temperature was regulated at 37°C, with 100 rpm stirring and no aeration. pH was regulated at 5.7 during the culture. After 16 hours, the cultures (OD at 700 nm culture 2.30) were inactivated by heat treatment at 65°C for 35 minutes and transferred to a harvest tank. Once inactivated, the cultures were transferred to an ultrafiltration skid in order to separate the biomass from the culture medium, concentrated, and washed with NaCl (9 g/L) in purified water. The harvested biomass was aliquoted (mass of concentrated bacterial suspension 2000 g at 31.8 mg dry weight biomass per gram of concentrated bacterial suspension) and then frozen at -15°C.

Example 1.2: Culture of *Lactobacillus helveticus* 103146

Initial Culture Conditions

Culture media was prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid: 1.1 mg/L): 0.5 mL/L. Polypropylene glycol (0.02 mL/L) was then added. After dissolution, the pH was not adjusted. After sterilizing the media, small Erlenmeyer flasks were individually inoculated with the content of frozen vials (containing 1.5 mL of frozen bacteria) and incubated at 37°C for 9 hours. Then 20 ml aliquots of this culture were transferred to larger Erlenmeyer flasks containing 1000 mL of culture media, and incubated again under the same conditions. After 15 hours, growth, the content of the 1 liter Erlenmeyer flask was transferred to the prefermenter.

Culture Conditions in Prefermenters

20 liters of culture media were prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid: 1.1 mg/L): 0.5 mL/L. Polypropylene glycol (0.02 mL/L) was then added. After dissolution, the pH was not adjusted. Incubation temperature was regulated at 37°C, with 100 rpm stirring and no aeration. The pH was not regulated during the culture. After 9 hours, 7 liters from the prefermenters were transferred to a fermenter. (OD at 700 nm prefermenter culture after 9 hours: 0.14). The cultures of the prefermenters were transferred under sterile conditions into fermenters.

Culture Conditions in Fermenters

70 liters of culture media were prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid: 1.1 mg/L): 0.5 mL/L. Polypropylene glycol (0.02 mL/L) was then added. After dissolution, the pH was not adjusted.
After sterilization, 8 g/L glucose was added to the culture. The incubation temperature was regulated at 33°C, with 100 rpm stirring and no aeration. pH was adjusted to 6.7 at the beginning of the culture with $CH_3COOH$. After 24 hours the cultures (OD at 700 nm culture 4.17) were inactivated by heat treatment at 65°C for 35 minutes and transferred to a harvest tank. Once inactivated, the cultures were transferred to an ultrafiltration skid in order to separate the biomass from the culture medium, concentrated, and washed with NaCl (9 g/L) in purified water (9 g/L). The harvested biomass was aliquoted (mass of concentrated bacterial suspension 440 g at 19.1 mg dry weight biomass per gram of concentrated bacterial suspension) and then frozen at -15°C.

Example 1.3: Culture of *Lactobacillus plantarum* 71.39

Initial Culture Conditions

Culture media was prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid: 1.1 mg/L): 0.5 mL/L. Polypropylene glycol (0.02 mL/L) was then added. After dissolution, the pH was not adjusted. After sterilizing the media, small Erlenmeyer flasks were individually inoculated with the content of frozen vials (containing 1.5 mL of frozen bacteria) and incubated at 35°C for 9 hours. Then 20 ml aliquots of this culture were transferred to larger Erlenmeyer flasks containing 1000 mL of culture media, and incubated again under the same conditions. After 15 hours growth, the content of the 1 liter Erlenmeyer flask was transferred to the prefermenter.

Culture Conditions in Prefermenters

20 liters of culture media were prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L;

Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid: 1.1 mg/L): 0.5 mL/L. Polypropylene glycol (0.02 mL/L) was then added. After dissolution, the pH was not adjusted. The incubation temperature was regulated at 37°C, with 100 rpm stirring and no aeration. The pH was not regulated during the culture. After 9 hours, 7 liters from the prefermenters were transferred to a fermenter. (OD at 700 nm prefermenter culture after 9 hours = 1.62). The cultures of the prefermenters were transferred under sterile conditions into fermenters.

Culture Conditions in Fermenters

70 liters of culture media were prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid: 1.1 mg/L): 0.5 mL/L. Polypropylene glycol (0.02 mL/L) was then added. After dissolution, the pH was not adjusted.

After sterilization, 8 g/L glucose was added to the culture. The incubation temperature was regulated at 35°C, with 100 rpm stirring and no aeration. After 24 hours, the cultures (OD at 700 nm culture = 6.36) were inactivated by heat treatment at 65°C for 35 minutes and transferred to a harvest tank. Once inactivated, the cultures were transferred to an ultrafiltration skid in order to separate the biomass from the culture medium, concentrated, and washed with NaCl (9 g/L) in purified water. The harvested biomass was aliquoted (mass of concentrated bacterial suspension 600 g at 60.7 mg dry weight biomass per gram of concentrated bacterial suspension) and then frozen at -15°C.

Example 1.4: Culture of *Lactobacillus rhamnosus* 71.38

Initial Culture Conditions

Culture media was prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid: 1.1 mg/L): 0.5 mL/L. After dissolution, the pH was not adjusted. After sterilizing the media, small Erlenmeyer flasks were individually inoculated with the content of frozen vials (containing 1.5 mL of frozen bacteria) and incubated at 35°C for 9 hours. Then 20 ml aliquots of this culture were transferred to larger Erlenmeyer flasks containing 1000 mL of culture media, and incubated again under the same conditions. After 15 hours growth, the content of the 1 liter Erlenmeyer flask was transferred to the prefermenter.

Culture Conditions in Prefermenters

20 liters of culture media were prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid : 1.1 mg/L): 0.5 mL/L. Polypropylene glycol (0.02 mL/L) was then added. After dissolution, the pH was not adjusted. The incubation temperature was regulated at 35°C, with 100 rpm stirring and no aeration. The pH was not regulated during the culture. After 9 hours, 7 liters from the prefermenters were transferred to a fermenter. (OD at 700 nm prefermenter culture after 9 hours: 3.75). The cultures of the prefermenters were transferred under sterile conditions into fermenters.

Culture Conditions in Fermenters

70 liters of culture media were prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid: 1.1 mg/L): 0.5 mL/L. Polypropylene glycol (0.02 mL/L) was then added. After dissolution, the pH was not adjusted.

After sterilization, 8 g/L glucose was added to the culture. The incubation temperature was regulated at 35°C, with 100 rpm stirring and no aeration. After 14 hours the cultures (OD at 700 nm culture 5.43) were inactivated by heat treatment at 65°C for 35 minutes and transferred to a harvest tank. Once inactivated, the cultures were transferred to an ultrafiltration skid in order to separate the biomass from the culture medium, concentrated, and washed with NaCl (9 g/L) in purified water. The harvested biomass was aliquoted (mass of concentrated bacterial suspension 2798 g at 51.7 mg dry weight biomass per gram of concentrated bacterial suspension) and then frozen at -15°C.

Example 1.5: Culture of *Lactobacillus johnsonii* 103782

Initial Culture Conditions

Culture media was prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate : 0.2 g/L; Metal solution (copper sulfate: 3 mg/l; iron chloride: 830 mg/l; zinc sulfate: 860 mg/l; sulfuric acid : 1.1 mg/L): 0.5 mL/L. Polypropylene glycol (0.02 mL/L) was then added. After dissolution, the pH was not adjusted. After sterilizing the media, small Erlenmeyer flasks were individually inoculated with the content of frozen vials (containing 1.5 mL of frozen bacteria) and incubated at 33°C for 10 hours. Then 20 ml aliquots of this culture were transferred to larger Erlenmeyer flasks containing 1000 mL of culture media, and incubated again in the same conditions. After 14 hours growth, the content of the 1 liter Erlenmeyer flask was transferred to the prefermenter.

Culture Conditions in Prefermenters

20 liters of culture media was prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate : 0.2 g/L; Metal solution (copper sulfate: 3 mg/l; iron chloride: 830 mg/l; zinc sulfate: 860 mg/l; sulfuric acid : 1.1 mg/L): 0.5 mL/L. Polypropylene glycol (0.02 mL/L) was then added. After dissolution, the pH was not adjusted. Incubation temperature was regulated at 35°C, with 100 rpm stirring and no aeration. The pH of the culture was adjusted to 5.6 with acetic acid. After 24 hours, 7 liters from the prefermenters were transferred to a fermenter. (OD at 700 nm prefermenter culture after 24 hours: 0.47). The cultures of the prefermenters were transferred under sterile conditions into fermenters.

Culture Conditions in Fermenters

70 liters of culture media were prepared by dissolving in purified water the following components: Sodium chloride: 3 g/L; Sodium monohydrogen phosphate: 2 g/L; Sodium acetate: 1 g/L; Soya peptone 50 g/L; Glucose: 12 g/L; Calcium chloride: 0.1 g/L; Potassium chloride: 0.1 g/L; Sodium bicarbonate: 0.5 g/L; pyruvate: 0.1 g/L; Glutamate: 0.2 g/L; Metal solution (copper sulfate: 3 mg/l; iron chloride: 830 mg/l; zinc sulfate: 860 mg/l; sulfuric acid: 1.1 mg/L): 0.5 mL/L. Polypropylene glycol (0.02 mL/L) was then added. After dissolution, the pH was not adjusted.
After sterilization, 8 g/L glucose was added to the culture. The incubation temperature was regulated at 35°C, with 100 rpm stirring and no aeration. After 24 hours the cultures (OD at 700 nm, 0.174) were inactivated by heat treatment at 70°C for 30 minutes and transferred to a harvest tank. Once inactivated, the cultures were transferred to an ultrafiltration skid in order to separate the biomass from the culture medium, concentrated, and washed with NaCl (9 g/L) in purified water. The harvested biomass was aliquoted (mass of concentrated bacterial suspension 61.5 g at 20.2 mg dry weight biomass per gram of concentrated bacterial suspension) and then frozen at -15°C.

**Example 2: Bacterial Lysates**

Example 2.1

One aliquot of *Lactobacillus fermentum* I-3929 biomass from Example 1.1 containing 6 g of bacterial dry weight was thawed to room temperature, then diluted with purified water to reach 12 g/L of bacterial biomass dry weight. Alkalinization was performed with 0.03 M sodium hydroxide. The pH measured at the beginning of the lysis was 10.3. Then the lysis was incubated for 6 hours at 40°C under continuous stirring. After the incubation, the pH was 9.9.

Example 2.2

One aliquot of *Lactobacillus rhamnosus* 71.38 biomass from Example 1.4 containing 20 g of bacterial dry weight was thawed to room temperature, then diluted with purified water to reach 40 g/L of bacterial biomass dry weight. Alkalinization was performed with 0.03 M sodium hydroxide. The pH measured at the beginning of the lysis was 10.3. Then the lysis was incubated for 6 hours at 40°C under continuous stirring. After the incubation, the pH was 9.7.

Example 2.3

One aliquot of *Lactobacillus fermentum* I-3929 biomass from Example 1.1 containing 6 g of bacterial dry weight was thawed to room temperature, then diluted with purified water to reach 12 g/L of bacterial biomass dry weight. Alkalinization was performed with 0.06 M sodium hydroxide. The pH measured at the beginning of the lysis was 12.3. Then the lysis was incubated for 6 hours at 40°C under continuous stirring. After the incubation, the pH was 11.8.

Example 2.4

One aliquot of *Lactobacillus fermentum* I-3929 biomass from Example 1.1 containing 6 g of bacterial dry weight was thawed to room temperature, then diluted with 0.2 N NaCl solution to reach 12 g/L of bacterial biomass dry weight. The final concentration of NaCl solution was 0.15N. The pH measured at the beginning of the lysis was 6.4. Then the lysis was incubated for 6 hours at 40°C under continuous stirring. After the incubation, the pH was 6.3.

Example 2.5

An aliquot of the lysate of Example 2.1 was taken to continue the lysis process. The volume was adjusted at pH 3.6 with HCl 25% and then incubated in a water-bath at 40°C for 1 hour under static conditions.

Example 2.6

An aliquot of the lysate of Example 2.4 was taken to continue the lysis process. The volume was adjusted at pH 12.4 with NaOH 10N and then incubated in a water-bath at 40°C for 1 hour under static conditions.

Example 2.7

One aliquot of *Lactobacillus plantarum* 71.39 biomass from Example 1.3 containing 0.4 g of bacterial dry weight was thawed to room temperature then diluted with purified water to reach 7 g/L of bacterial biomass dry weight. Alkalinization was performed with 0.06 M sodium hydroxide. The pH measured at the beginning of the lysis was 12.6. Then the lysis was incubated for 2 hours at 40°C under continuous stirring. After the incubation, the pH was 12.4.

Example 2.8

One aliquot of *Lactobacillus johnsonii* 103782 biomass from Example 1.5 containing 0.3 g of bacterial dry weight was thawed to room temperature then diluted with purified water to reach 6 g/L of bacterial biomass dry weight. Alkalinization was performed with 0.06 M sodium hydroxide. The pH measured at the beginning of the lysis was 12.5. Then the lysis was incubated for 2 hours at 40°C under continuous stirring. After the incubation, the pH was 12.3.

Example 2.9

One aliquot of *Lactobacillus helveticus* 103146 biomass from Example 1.2 containing 0.4 g of bacterial dry weight was thawed at room temperature, then diluted with purified water to reach 8 g/L of bacterial biomass dry weight. Alkalinization was performed with 0.06 M sodium hydroxide. The pH measured at the beginning of the lysis was 12.8. Then the lysis was incubated for 2 hours at 40°C under continuous stirring. After the incubation, the pH was 12.2.

Example 2.10

One aliquot of *Lactobacillus fermentum* I-3929 biomass from Example 1.1 containing 6.8 g of bacterial dry weight was thawed at room temperature, then diluted with purified water to reach 7 g/L of bacterial biomass dry weight. Alkalinization was performed with 0.08 M sodium hydroxide. The pH measured at the beginning of the lysis was 12.2. Then the lysis was incubated for 7 hours at 40°C under continuous stirring. After the incubation, the pH was 12.0. The lysis pH was adjusted to 9.8 with HCl.
The lysis comprised solubilised dry weight (SDW): 20.5 mg/g, proteins (Prot): 4.9 mg/g.

Example 2.11

One aliquot of *Lactobacillus fermentum* I-3929 biomass from Example 1.1 containing 6.8 g of bacterial dry weight was thawed to room temperature then diluted with purified water to reach 7 g/L of bacterial biomass dry weight. Alkalinization was performed with 0.15 M sodium hydroxide. The pH measured at the beginning of the lysis was 13.0. Then the lysis was incubated for 63 hours at 40°C under continuous stirring. After the incubation, the pH was 12.6. The lysis pH was adjusted to 9.9 with HCl.

The lysis comprised SDW: 23.7 mg/g, Prot: 5.0 mg/g.

Example 2.12

One aliquot of *Lactobacillus fermentum* I-3929 biomass from Example 1.1 containing 98 g of bacterial dry weight was thawed to room temperature, then diluted with purified water to reach 10 g/L of bacterial biomass dry weight. Alkalinization was performed with 0.08 M sodium hydroxide. The pH measured at the beginning of the lysis was 12.0. Then the lysis was incubated for 24 hours at 40°C under continuous stirring. After the incubation, the pH was 11.1. The lysis pH was adjusted to 11.5 with NaOH.

The lysis comprised SDW: 23.7 mg/g.

Example 2.13

One aliquot of *Lactobacillus fermentum* I-3929 biomass from Example 1.1 containing 39 g of bacterial dry weight was thawed to room temperature, then diluted with a 0.2N NaCl solution to reach 7.6 g/L of bacterial biomass dry weight in order to produce an osmotic lysate. The pH measured at the beginning of the lysis was 4.4. Then the bacterial osmotic lysat was incubated for 24 hours at 40°C under continuous stirring. After the incubation, the pH was 4.2. The bacterial lysate was then made alkaline with addition of NaOH to reach 0.06 N. The pH measured at the beginning of the alkaline lysis was 10.6. The lysis was re-incubated for 2.25 hours at 40°C under continuous stirring. After the incubation, the pH was 10.2.

**Example 3: Purification of Lysates**

Example 3.1

The lysate of Example 2.5 was centrifuged for 20 minutes at 3000 x g. Then the supernatant was adjusted to pH 6.8 with NaOH 10N and filtered through successive filters with porosities of 0.45 $\mu$m and 0.2 $\mu$m, and finally with a sterile filter 0.22 $\mu$m. The concentrate comprised Prot: 2.8 mg/mL; DNA: 17.4 $\mu$g/mL. D-Amino acid percentage: 14.9% D-Ala, 14.4% D-Pro, 14.2% D-Asp.

NO production in mg of dry weight /mL: 0.003 mg/mL (C1) — 0.03 mg/mL (C2) — 0.3 mg/mL (C3) of active dry weight /mL: C1: 3.3 $\mu$M, C2: 33.2 $\mu$M, C3: 52.5 $\mu$M. Active Dry Weight in g/L or mg/mL is the soluble dry weight in g/L or mg/mL minus the chloride content in g/L or mg/mL.

Example 3.2

The lysate of Example 2.6 was centrifuged for 20 minutes at 3000 x g. Then the supernatant was adjusted to pH 7 with HCl and filtered through successive filters with porosities of 0.45 $\mu$m and 0.2 $\mu$m, and finally with a sterile filter 0.22 $\mu$m. The concentrate comprised Prot: 12.3 mg/mL; DNA: 27.7 $\mu$g/mL. D-Amino acid percentage: 15.9% D-Ala, 24.4% D-Pro, 17.4% D-Asp, 45.1 % D-Ser, 10.1 % D-Met.

NO production in mg of active dry weight /mL: 0.003 mg/mL (C1) — 0.03 mg/mL (C2) — 0.3 mg/mL (C3) of active dry weight /mL: C1: 8.0 $\mu$M, C2: 56.0 $\mu$M, C3: 94.3 $\mu$M.

Example 3.3

300 ml of the lysate of Example 2.1 was first centrifuged at 3000 x g for 20 minutes. The supernatant was adjusted to pH 7.1 with HCl. The extract was concentrated through a 0.45 $\mu$m membrane in polythersulfone (PES) (Sartorius Stedim Biotech GmbH) with a constant flow at 330-350 mL/min on a Sartoflow® Slice 200 Benchtop Crossflow System. The yield volume was 75%. The concentrate was finally sterile filtered through a PES membrane 0.2 $\mu$m (Nalgene).

The concentrate comprised SDW: 30.0 mg/g; Prot: 9.6 mg/mL; DNA: 32.8 $\mu$g/mL. D-Amino acid percentage: 14.3% D-Ala, 13.9% D-Pro, 14.1% D-Asp, 36.9% D-Ser. NO production in mg of active dry weight /mL: 0.003 mg/mL (C1) — 0.03 mg/mL (C2) — 0.3 mg/mL (C3) of active dry weight /mL: C1: 5.1 $\mu$M, C2: 30.0 $\mu$M, C3: 64.0 $\mu$M.

Example 3.4

300 ml of the lysate of Example 2.2 was adjusted to pH 7.0 with HCl. The extract was filtered as described in Example 3.3 with a constant flow at 350 mL/min. The yield volume was more than 75%. The concentrate was finally sterile filtered through a PES membrane 0.2$\mu$m (Nalgene).

The concentrate comprised SDW: 49.2 mg/g; Prot: 14.2 mg/mL; DNA: 34.1 $\mu$g/mL. D-Amino acid percentage: 16.0% D-Ala, 13.3% D-Pro, 14.2% D-Asp.

NO production in mg of active dry weight /mL: 0.003 mg/mL (C1) — 0.03 mg/mL (C2) — 0.3 mg/mL (C3) of active dry weight /mL: C1: 0 $\mu$M, C2: 4.1 $\mu$M, C3: 26.3 $\mu$M.

Example 3.5

300 ml of the lysate of Example 2.3 was first centrifuged at 3000xg for 20 minutes. The supernatant was adjusted to pH 7.1 with HCl. The extract was filtered as described in Example 3.3 with a constant flow at 330-350 mL/min. The yield volume was more than 75%. The concentrate was finally sterile filtered through a PES membrane 0.2 $\mu$m (Nalgene).

The concentrate was composed of SDW: 34.5 mg/g; Prot: 8.9 mg/mL; DNA: 16.8 $\mu$g/mL. D-Amino acid percentage: 16.4% D-Ala, 24.3% D-Pro, 17.3% D-Asp.

NO production in mg of active dry weight /mL: 0.003 mg/mL (C1) — 0.03 mg/mL (C2) — 0.3 mg/mL (C3) of active dry weight /mL: C1: 3.0 $\mu$M, C2: 36.4 $\mu$M, C3: 69.5 $\mu$M.

Example 3.6

1000mL of the lysate of Example 2.13 was centrifuged for 20 minutes at 9384 x g. The supernatant was filtered directly through a sterile filter 0.22 $\mu$m.

The concentrate comprised SDW: 32.7 mg/g; Prot: 6.5 mg/g; Sugar: 2.4 mg/g. The sugar concentration was assayed according to the procedure by Herbert et al. (Meth. Microbiol, 1971, 5B:266 et seq.).

Screening of biological activity was performed on human Peripheral Blood Mononuclear Cells (PBMC) at different concentrations as described in Example 6. Results obtained at 0.5 mg of active dry weight /mL were TNF-$\alpha$: 83 pg/mL; IL-6: 898 pg/mL; IL-12: 140 pg/mL (in the presence of 10 ng/ml IFN-$\gamma$); and IL-10: 221 pg/mL (in the presence of IFN-$\gamma$).

Example 3.7

The bacterial lysate mixture of Example 2.10 was transferred into a microfiltration (MF) tank after being adjusted to pH 10.2. The microfiltration (MF) unit used a 0.45 $\mu$m tangential flow filtration (TFF) filter (Sartocon Slice Sartorius). The cross flow was adjusted to 290 L/h m$^2$ (LHM) and the Trans Membrane Pressure (TMP) to 0.4-0.5 bar. The permeate was transferred to an ultrafiltration (UF) tank.

Once the volume of the lysate in the microfiltration tank had reached one-half of the initial volume, the UF unit was started. The permeate of UF filters was used as washing buffer in MF tank. The volumes of both the MF and UF tanks were maintained at the same level. After 10 diafiltration volumes, the UF was stopped, and the bacterial lysate was concentrated in the MF tanks. The recovered volumeric yield was 83%. The recovered extract in UF tank, was adjusted to pH 7.3 with HCl and then filtered through a 0.2 $\mu$m sterile filter.

The concentrate comprised SDW: 17.5 mg/g; Prot: 4.3 mg/g.

Example 3.8

The lysate of Example 2.7 was centrifuged for 15 minutes at 9384 x g. Then the supernatant was adjusted to pH 7.3 with HCl and filtered through successive filters with porosities of 0.45 $\mu$m and 0.2 $\mu$m, and finally with a sterile filter 0.22 $\mu$m.

Peripheral Blood Mononuclear Cells (PBMC) with a 10 times diluted product: 777 pg/mL IL-10, 26 pg/mL IL-12, 13183 pg/mL IL-6.

Example 3.9

The lysate of Example 2.8 was centrifuged for 15 minutes at 9384 x g. Then supernatant was adjusted to pH 7.4 with HCl and filtered through successive filters with porosities of 0.45 $\mu$m and 0.2 $\mu$m, and finally with a sterile filter 0.22$\mu$m.

PBMC with 10 times diluted product: 904 pg/mL IL-10, 0 pg/mL IL-12, 4995 pg/mL IL-6.

Example 3.10

The lysate of Example 2.9 was centrifuged for 15 minutes at 9384 x g. Then the supernatant was adjusted to pH 7.6 with HCl and filtered through successive filters with porosities of 0.45 $\mu$m and 0.2 $\mu$m, and finally with a sterile

filter 0.22μm.

PBMC with 10 times diluted product: 476 pg/mL IL-10, 0 pg/mL IL-12, 4924 pg/mL IL-6.

Example 3.11

The lysate of Example 2.11 was transferred to an MF tank after being adjusted to pH 10.4. The TFF installation was similar to Example 3.7. The cross flow was adjusted to 290 L/h m$^2$ and the TMP to 0.4-0.5 bar. The UF was stopped after 10 diafiltration volumes. The recovered volume yield was 86%. The final product was adjusted to pH 7.3 with HCl.

The concentrate obtained comprised SDW: 24.2 mg/g; Prot: 4.8 mg/g.

Example 3.12

The lysate of Example 2.12 was transferred to an MF tank after being adjusted to pH 11.5. The TFF installation was similar to Example 3.7. The cross flow was adjusted to 290 L/h m$^2$ and the TMP to 0.4 bar. The UF was stopped after 10 diafiltration volumes. The recovered volume yield was 74%. The final product was adjusted to pH 7.2 with HCl.

The concentrate obtained comprised SDW: 32.4 mg/g; Prot: 5.8 mg/g; Sugar: 2.3 mg/g.

Screening of biological activity was performed on human Peripheral Blood Mononuclear Cells (PBMC) at different concentrations as described in Example 6. Results obtained at 0.5 mg of active dry weight /mL were TNF-α: 37 pg/mL; IL-6: 1092 pg/mL; IL-12: 81 pg/mL (in the presence of 10 ng/ml IFN-γ); and IL-10: 160 pg/mL (in the presence of 10 ng/ml IFN-γ). Protein concentration was measured by the DC Protein assay (BioRad, DC Protein assay, kit no. 500-0116). The microplate assay protocol was performed. Samples to analyze (0.1 mL) were diluted with 1.9 mL of 25 mM phosphate buffer pH 11. A protein standard curve was prepared each time the assay was performed with a bovine serum albumine solution at 2 mg BSA /ml (BSA, Pierce ref 23210). BSA solution was diluted with 25 mM phosphate buffer pH 11 to obtain the following concentrations: 0.18, 0.24, 0.3, 0.36 and 0.42 mg BSA/ml. Samples (20 μl) and BSA standards (20 μl) were added in quadruplicate into a clean dry microtiter plate. Reagent A (25 μl) from the DC Protein assay kit was added into each well. After 10 minutes, 200 μL of reagent B was added into each well. The microtiter plate was mixed on a rotary plate shaker for 5 seconds. After 20 minutes at room temperature the absorbance at 750 nm was recorded. The protein concentration of each sample was calculated using the slope of the linear regression on the protein standard curve:

$$\text{OD at 750 nm} = a * (\text{protein concentration}) + b$$

$$\text{mg protein in sample} = [20 \times (\text{OD at 750 nm} - b)] / a$$

The soluble dry weight (SDW) was determined by obtaining 5 mL of the soluble fraction resulting from the lysis and drying it to a constant mass in a porcelain dish at 105°C.

## Example 4: Activation of Murine Spleen Cells *In Vitro*

The immunostimulating effect of the embodiments of the invention was assayed *in vitro* by measuring the activation of Murine Spleen Cells (Alamar Blue assay).

Materials and Methods

Stimulation of spleen cells

The AlamarBlueTM-Assay is designed to measure quantitatively the growth of human or animal cells. This assay incorporates a fluorometric/colorimetric growth indicator based on the detection of metabolic activity by an oxidation-reduction (REDOX) indicator in response to chemical reduction resulting from cell growth. Related to growth, the REDOX indicator causes changes from the oxidized (non-fluorescent, blue) form to the reduced (fluorescent, red) form.

Mice Balb/c mice (female, 6-8 weeks of age) were received from Charles River Laboratories, Sulzfeld, Germany, and were sacrificed by cervical dislocation. Spleens were homogenized using a potter; cell suspensions were washed by centrifugation (280 x g, 4°C, 10 min) and resuspended in 5 ml RPMI 1640 containing 5% FCS, 100 U/ml penicillin and 100 μg/ml streptomycin. 100 μl spleen cells (2 x 10$^6$ /ml) were incubated with 50 μl of bacterial extract dilutions in 96-well plates (Falcon 3072) for 48 h at 37°C and 5 % CO$_2$ in cell culture medium.

After the addition of 30 μl AlamarBlueTM solution diluted 1:1 with culture medium, the chemical reduction of AlamarBlueTM was measured with a Fluoroskan Ascent Reader (ThermoLabsystems, Frankfurt, Germany) at 544 nm excitation and 590 nm emission wavelengths.

Test articles

The following extracts were tested:
Afer300: extract from *Lactobacillus fermentum 1-3929* (12 g/L) obtained as described in Example 3.5 (31.6 mg of active dry weight /g);
Cfer300: extract from *Lactobacillus fermentum 1-3929* (12 g/L) obtained as described in Example 3.3 (28.4 mg of active dry weight /g);
Dfer300: extract from *Lactobacillus fermentum 1-3929* (12 g/L) obtained as described in Example 2.4 and purified using the same conditions as Example 3.3 (29.5 mg of active dry weight /g). This example was used as a non-alkaline lysed control.
ARahr300: extract from *Lactobacillus rhamnosus* 71.38 (40 g/L) obtained by an alkaline lysis with NaOH 0.03 M at 40°C for 6 hours (49.3 mg of active dry weight /g);
CRahr300: extract from *Lactobacillus rhamnosus* 71.38 (40 g/L) obtained as described in Example 3.4 (46.4 mg of active dry weight /g);
DRahr300: extract from *Lactobacillus rhamnosus* 71.38 (40 g/L) lysed by osmotic stress in 0.15 N NaCl solution at 40 °C for 6 hours (46.2 mg of active dry weight /g). This example was used as a non-alkaline lysed control.

Neᵍative controls

Aqua dest. (distilled water) or phosphate buffered saline (PBS).

Results

*In vitro* studies to determine splenocyte activation

The increase of metabolic activity of mouse spleen cells after treatment with the extract was determined in 2 independent experiments by the Alamar Blue assay (see Figures 4a and 4b).
Spleen cells were cultured for 48 h in the presence of the extract. After the addition of Alamar blue® solution, emission values were measured. As shown in Figures 4a-b, the bacterial extracts were effective at dilutions around 1: 300. All groups were compared statistically to the control groups using student's T-test.

Table 1: p-value for bacterial extracts in 2 independent experiments by the Alamar Blue assay (see Figures 4a and 4b).

| Batch | Dilution | p-value test 1 | p-value test 2 |
|---|---|---|---|
| Afer300 | (dilution 1:300) | 0.000016 (Fig. 4a) | 0.00016 (Fig. 4b) |
| Cfer300 | (dilution 1:300) | 0.00021 (Fig. 4a) | 0.048 (Fig. 4b) |
| Dfer300 | (dilution 1:300) | 0.00033 (Fig. 4a) | 0.00016 (Fig. 4b) |
| ARahr300 | (dilution 1:300) | 0.0029 (Fig. 4a) | 0.0012 (Fig. 4b) |
| CRahr300 | (dilution 1:300) | 0.0031 (Fig. 4a) | 0.12 (Fig. 4b), |
| DRahr300 | (dilution 1:300) | 0.0025 (Fig. 4a) | 0.08 (Fig. 4b). |

Conclusion

The immunostimulating effect of bacterial extracts was assayed in *vitro* by measuring the activation of murine spleen cells by the Alamar Blue assay. Here, in two independent experiments, we compared an extract obtained by osmotic lysis (Dfer300), which includes intact, particulate cell wall components, with two extracts according to this invention (AFer300 and CFer300). Those three extracts were obtained from *Lactobacillus fermentum* I-3929. The AFer300, CFer300, and DFer300 extracts were each effective in stimulating the metabolism of the cells at a dilution of 1:300. We observed no difference between the DFer300 osmotic lysis control and the Afer300 and CFer300.
We also compared three extracts obtained from a second strain, *Lactobacillus rhamnosus* 71.38 (ARahr300, CRahr300, DRahr300). As in the previous set, ARahr300 and CRahr300 are within the scope of the invention while DRahr is an osmotic lysis control. Here, the ARahr300 extract was effective in two independent assays at a dilution of 1:300, whereas the CRahr300 and DRahr300 extracts showed a weaker but significant stimulation in one of the two assays at the same dilution. In comparing the results of all six extracts, it also appears that the *Lactobacillus fermentum* I-3929 extracts were more effective in stimulating spleen cell growth in comparison to the *Lactobacillus rhamnosus* 71.38 extracts.

**Example 5: Production of Nitric Oxide by Bone Marrow-Derived Macrophages**

The immunotimulating potential of a series of embodiments according to the invention was tested by measuring the production of nitric oxide (NO) by murine bone marrow-derived macrophages.

Materials and Methods

Six-week old male C57/BL6 mice (six weeks old male, SPF quality, Charles Rivier, FR) were killed by $CO_2$ inhalation. The hip, femur, and tibia from the posterior appendage were removed. The bone marrow was extracted from the lumen by injecting Dulbecco's Modified Eagle Medium (DH) through the bone after cutting both end portions. After washing, the stem cells were resuspended (40000 cells/mL) in DH medium supplemented with 20% horse serum and 30% L929 cell supernatant. The cell suspension was incubated for 8 days in an incubator at 37°C under 8% $CO_2$ and moisture-saturated atmosphere. Macrophages were then detached with ice-cold PBS, washed and resuspended in DH medium supplemented with 5% fetal calf serum (FCS), amino acids and antibiotics (DHE medium). The cell density was adjusted to 700000 cells/mL. Aqueous solutions of the extracts were serially diluted in DHE medium directly in microtiter plates. The extracts of the invention were tested in triplicates and each microtiter plate comprised the medium as negative control. The final volume in each well was 100 µL. 100 µL of the cell suspension was added to the diluted extracts and the cells were incubated for 22 hours in an incubator at 37°C, under 8% $CO_2$ and a moisture-saturated atmosphere. At the end of the incubation period, 100 µL of supernatant was transferred to another microtiter plate and the nitrite concentration produced in each supernatant was determined by running a Griess reaction. 100 µL of Griess reagent (5 mg/mL of sulfanilamide + 0.5 mg/mL of N-(1-naphtyl)ethylene-diamine hydrochloride) in 2.5% aqueous phosphoric acid, was added to each well. The microtiter plates were read with a spectrophotometer (SpectraMax Plus, Molecular Devices) at 562 nm against a reference at 690 nm. The nitrite concentration was proportional to nitric oxide content being formed. The nitrite content was determined based on a standard curve of sodium nitrite (1 to 70 µM $NaNO_2$). The results were given in µM nitric oxide (NO) as mean value ± standard deviation and plotted as a dose response curve.

Test articles

The following extracts were tested:

First assay:

OP0701 B4_CFer300: extract from *Lactobacillus fermentum 1-3929* (12 g/L) obtained as described in Example 3.3;

OP0701 B4_Dfer300: extract from *Lactobacillus fermentum 1-3929* (12 g/L) obtained as described in Example 2.4 and purified using the same conditions as Example 3.3. This example was used as a non-alkaline lysed control.

OP0701 B4_CRahr300: extract from *Lactobacillus rhamnosus* 71.38 (40 g/L) obtained as described in Example 3.4

OP0701 B4_DRahr300: extract from *Lactobacillus rhamnosus* 71.38 (40 g/L) obtained by osmotic stress in 0.15 N NaCl solution at 40 °C for 6hours. This example was used as a non-alkaline lysed control.

Second assay:

OP0701C_10G0.5P4H: extract from *Lactobacillus fermentum 1-3929* at 10 g/l of biomass dry weight with 0.037M NaOH incubated at 40°C for 4 hours.

OP0701C_10G1P4H: extract from *Lactobacillus fermentum 1-3929* at 10 g/l of biomass dry weight with 0.075M NaOH incubated at 40°C for 4 hours.,

OP0701C_10G2P4H: extract from *Lactobacillus fermentum 1-3929* at 10 g/l of biomass dry weight with 0.150 M NaOH incubated at 40°C for 4 hours.,

OP0701C_10G1P21H: extract from *Lactobacillus fermentum 1-3929* at 10 g/l of biomass dry weight with 0.075M NaOH incubated at 40°C for 21 hours.

OP0701C_10G0.5P21H: extract from *Lactobacillus fermentum I-3929* at 10 g/l of biomass dry weight with 0.037M NaOH incubated at 40°C for 21 hours.,

OP0701C_10G2P21H: extract from *Lactobacillus fermentum 1-3929* at 10 g/l of biomass dry weight with 0.150 M NaOH incubated at 40°C for 21 hours.,

OP0701C_10G2P45H: extract from *Lactobacillus fermentum 1-3929* at 10 g/l of biomass dry weight with 0.150 M NaOH incubated at 40°C for 45 hours.

Results first assay

In the first assay (Figure 5a), the extracts obtained from alkaline lysis in accordance to the present invention were observed to have the same activity as the extracts obtained from an osmotic lysis.

We also observed that the immunostimulation activity was related to the strain selected. The extracts obtained from the *Lactobacillus fermentum* I-3929 induced greater $NO_2$ and $NO_3$ production than the extracts obtained from the strain *Lactobacillus rhamnosus* 78.31.

Results second assay

In the second assay (Figure 5b), we observed that the *in vitro* activity of the extract was correlated to the initial conditions of the lysis. The activities of the following bacterial extracts are shown for the same strain *Lactobacillus fermentum I*-3929, and for the same amount 10g of biomass dry weight / liter of lysis:

OP0701C_10G0.5P4H, OP0701C_10G1P4H, OP0701C_10G2P4H,

OP0701C_10G1P21H, OP0701C_10G0.5P21H, OP0701C_10G2P21H, and

OP0701C_1OG2P45H. The *in vitro* activity depended on the initial concentration of NaOH and the duration of the lysis.

Conclusion

Results of these experiments showed that despite the chemical modifications generated by the alkaline process, the activity of the embodiments is not decreased when compared to aa control extract obtained by osmotic lysis or compared to the respective live bacteria (see example 6 below for this aspect).

**Example 6: *In vitro* Screen for Pro- and Anti-Inflammatory Activity**

In order to screen embodiments of the invention for their immunostimulating or anti-inflammatory potential *in vitro,* tests were performed on a series of bacterial extracts on human PBMCs. The release of both IL12p70 (an inflammatory cytokine) and IL10 (an anti-inflammatory cytokine) was measured, and the IL-10/IL-12 ratio was reported, as described by Foligne et al. (World J Gastroenterol 2007 January 14; 13(2): 236-243).

The aim was to compare the production of IL-12p70 and IL-10 on a serie of 6 extracts obtained via different extraction/purification methods. The IL-10/IL-12p70 ratio obtained for each extract may correlate with the anti-inflammatory potential of the extracts. Live bacteria was used as a control against which to compare the activities of the extracts ($2x10^7$ cfu/ml of *Lactobacillus fermentum* I-3929).

Materials and Methods

Graded amounts of the 6 bacterial extracts were diluted in cell culture medium in the presence of 10 ng/ml of IFN-γ, a cytokine known to enhance the production of IL-12p70, starting with an initial dose of 1 mg/ml (highest final dose tested). PBMCs isolated from the blood of healthy donors were tested as serial dilutions of active dry weight extracts from 100 ng/ml to 1 mg/ml.

IFN-γ was added at least 3 hours before the lysates or the live bacterial strain in the medium. Data from two independent experiments are reported.

Preparation of human PBMCs

PBMCs were isolated from peripherical blood. Briefly, after a Ficoll gradient centrifugation (Pharmacia, Uppsala, Sweden), mononuclear cells were collected, washed in RPMI 1640 medium (Live technologies, Paisley, Scotland) and adjusted to $2 \times 10^6$ cells/mL in RPMI 1640 supplemented with gentamicin (150 μg/mL), L-glutamine (2 mmol/L), and 10% foetal calf serum (FCS) (Gibco-BRL).

Induction of cytokines

PBMCs (2 × 10⁶ cells/mL) were seeded in 24-well tissue culture plates (Corning, NY). Cells were stimulated as described above. After 24 hours stimulation at 37°C in an atmosphere of air with 5% $CO_2$, the culture supernatants were collected, clarified by centrifugation and stored at -20°C until cytokine analysis. Cytokines were measured by ELISA using pharmingen antibody pairs (BD Biosciences, San Jose, Ca, USA) for IL-10 and IL-12p70, according to the manufacturer's recommendations.

Test articles

OP0701 C_1 0G2P45H (A): extract from *Lactobacillus fermentum* I-3929 lysis at 10g of biomass dry weight / liter of lysis, 0.15 M NaOH at 40°C for 45 hours.
OP0701C_10G1 P4H (B): extract from *Lactobacillus fermentum* I-3929 lysis at 10g of biomass dry weight / liter of lysis, 0.075 M NaOH at 40°C for 4 hours.
OP0701 C_5G4P21 H (C): extract from *Lactobacillus fermentum* I-3929 lysis at 5g of biomass dry weight / liter of lysis, 0.300 M NaOH at 40°C for 21 hours.
OP0701C_40G0.5P4H (D): extract from *Lactobacillus fermentum* I-3929 lysis at 40g of biomass dry weight / liter of lysis, 0.037 M NaOH at 40°C for 6 hours.
OP0701 B4_CFer150 (E): extract from *Lactobacillus fermentum I-3929* (12 g/L) obtained as described in Example 3.1
OP0701 B4_BFer300 (F): extract from *Lactobacillus fermentum* I-3929 lysis at 6 g of biomass dry weight / liter of lysis, 0.075 M NaOH at 40°C for 6 hours.
*Lactobacillus fermentum 1-3929,* live bacteria frozen at -80°C at 2x10⁷ cfu/ml in 20% glycerol.

Results

The aim of this test was to compare the *in vitro* immunostimulating or anti-inflammatory potential of embodiments of the invention with a live *Lactobacillus* strain. In order to analyze the different extracts and the live bacteria, the quantity of each cytokine IL-10 and IL-12 released from PBMCs was compared, as well as the IL10/IL12 ratio.
If either the IL-10 or IL-12 concentrations, or both values, were close to the non-stimulated levels (i.e., below 10 pg/ml), the ratio was not considered calculable and labeled N.C. (non-calculated value).
Results obtained from the PBMCs expressed in pg/ml are shown in Tables 2 and 3.
In the presence of IFNγ, the effect of 6 bacterial extracts of the invention (Extract A to Extract F) were compared to a live form of *Lactobacillus fermentum* (2x10⁷ bacteria/ml, tested twice as Live sample 1 and Live sample 2) on human PBMCs (IL10 and IL12p70 responses in pg/ml). The ratios IL10/IL12 are reported only for the doses of extracts of 100 μg/ml and 1 mg/ml.

Table 2: *in vitro* pro-inflammatory (immunostimulating) or anti-inflammatory (immunomodulatory) potential of the bacterial extracts in human PBMCs.

| | A | B | C | D | E | F |
|---|---|---|---|---|---|---|
| **IL10 (+ IFNγ)** | | | | | | |
| 1mg/ml | 146 | 1089 | 9 | 953 | 961 | 549 |
| 100µg/ml | 3 | 232 | 1 | 121 | 106 | 6 |
| **IL12 (+ IFNγ)** | | | | | | |
| 1mg/ml | 8 | 78 | 1 | 94 | 77 | 48 |
| 100µg/ml | 1 | 110 | 1 | 59 | 162 | 11 |
| **IL10/IL12 (+ IFNγ)** | | | | | | |
| 1mg/ml | N.C. | 14.03 | N.C. | 10.17 | 12.49 | 11.40 |
| 100µg/ml | N.C. | 2.11 | N.C. | 2.05 | 0.65 | 0.52 |

N.C.: Non Calculable value

In the presence of IFNγ, the effect of 6 extracts of the invention (Extract A to Extract F) were compared to a live form of *Lactobacillus fermentum* (2x10⁷ bacteria/ml, tested twice as Live sample 1 and Live sample 2) on human PBMCs (IL10 and IL12p70 responses, in pg/ml). The ratios IL10/IL12 are reported only for the doses of extracts of 100

μg/ml and 1 mg/ml.

Table 3 : *in vitro* pro-inflammatory (immunostimulating) or anti-inflammatory (immunomodulatory) potential of the live *Lactobacillus fermentum* Strain I-3929 in human PBMCs.

|  | *Lactobacillus fermentum* I-3929 Live sample 1 | *Lactobacillus fermentum* I-3929 Live sample 2 |
|---|---|---|
| **IL10 (+ IFNγ)** | | |
| **2x10⁷ CFU/ml** | 386 | 355 |
| **IL12 (+ IFNγ)** | | |
| **2x10⁷ CFU/ml** | 187 | 191 |
| **IL10/IL12 (+ IFNγ)** | | |
| **2x10⁷ CFU/ml** | 2.06 | 1.86 |

Based on the results presented in Table 2 and Table 3, the production of IL10 was decreasing in the following order:

$$B \cong E \cong D > F > \text{live } \textit{Lactobacillus} > A > C$$

Based on the results presented in Table 2 and Table 3, the production of IL12 was decreasing in the following order:

$$\text{live } \textit{Lactobacillus} > E > B > D > F > A > C$$

When considering the ratio, the following general pattern was observed:

$$B \cong D > E \cong F > \text{live } \textit{Lactobacillus}$$

Results of IL10/IL12 ratios for extracts A and C are not shown since these extracts were not found to be effective inducers of cytokines.
At concentrations lower than 100 μg/ml, the cytokine concentrations observed were too low to draw any conclusions.

Conclusion

The ratios obtained could suggest that, under the experimental conditions used in Example 6, some bacterial extracts of the invention have greater immunomodulatory effects than those of live *Lactobacillus fermentum*. For example, extracts B, D, E, and F showed higher IL10/IL12p70 ratios than the live bacteria control. Thus, such extracts could be more active than live probiotic bacteria against conditions described herein, such as inflammatory conditions.

**Example 7: Action on Toll-Like Receptors**

Embodiments of the invention are obtained from gram positive bacteria, and therefore are expected to act via TLR2 receptors. TLR receptors are expressed principally, but not exclusively, by immune cells such as monocytes, macrophages, dendritic cell, T-cells etc, and are key sensors of microbial products, which can be recognized as signal dangers by the host. Even though they trigger first an unspecific innate immunity, TLR activation initiates a full immunological cascade which results, in the presence of antigens, to the development of acquired immunity.
Cells that express a given functional TLR gene are valuable tools for many applications, such as the study of the mechanisms involved in TLR recognition or signaling, and the development of new potential therapeutic drugs. The experiments described below tested the activity of three baterial extracts on these key adaptors of the immune response.

Materials and Methods

The responses of the extracts of the invention were tested (either *per* se to check their agonist effect or, in the presence of the TLR2 agonist Pam3Cys or in the presence of the TLR4 agonist LPS to check for antagonistic activities) in the two following cellular systems:

a) HEK-TLR2/6 (IL-8 ELISA after 24 hours)
b) HEK-MD2-TLR4-CD14 (IL-8 ELISA after 24 hours)

a) HEK-TLR2/6

The HEK293 cell line was chosen for its null or low basal expression of the TLR genes. These cells enable efficient monitoring of TLR activity using ELISA analysis such as IL-8 titration or reporter-based systems that monitor TLR-induced NF-κB activation.
HEK-TLR2/6 cells (Invivogen, Toulouse, France) are engineered HEK293 cells stably transfected with multiple genes from the TLR2/6 pathway that include TLR2, TLR6 and genes participating in the recognition or involved in the signaling cascade. These cells secrete IL-8 after TLR2/6 stimulation. Experiments were performed according to the manufacturer's instructions.
Briefly, $2x10^4$ cells/well (200ul RPMI) were incubated at 37°C during 3 days (5% $CO_2$). The medium was removed and 90 μl RPMi + 5 %FCS was added to the wells. Then the agonists and controls were added (10 μl/well). The cells were returned to the incubator for 24 hours. The supernatants were collected and the IL-8 ELISA was performed according to the manufacturer's instructions.

Test articles

OP0701 B4_Afer50: extract from *Lactobacillus fermentum* I-3929 lysis at 12g of biomass dry weight / liter of lysis, 0.075 M NaOH at 40°C for 4 hours, purified as described in Example 3.6.
OP0701 C-Bt1 LAC: extract from *Lactobacillus fermentum* I-3929 obtained as described in Example 3.7.
OP0701C-Bt2LAC: extract from *Lactobacillus fermentum* I-3929 obtained as described in Example 3.11.

Results: IL-8 secretion

The results for the controls (negative = LPS K12 ultrapure, TLR4 agonist; and PAM3CSK4 = positive, TLR2 agonist) are provided in Tables 4-6. The results (expressed as pg/ml of IL-8) show the mean values of IL-8 secretion determined by ELISA 24 hours after stimulation with the controls.
The cell line HEK TLR2/6 responded to the TLR2 agonist Pam3Cys. In contrast, the TLR 4 agonist from *E coli* (LPS K12) was inactive, even at the high dose of 0.01 μg/ml.

Experiments with the 3 bacterial extracts alone

The 3 bacterial extracts tested here (OP0701 B4 Afer50, OP0701 C-Bt1 LAC, and OP0701C-Bt2LAC) exhibited higher immunostimulating properties than the TLR2 agonist Pam3Cys.

Experiments with the 3 bacterial extracts + Pam3Cys

As mentioned above, the three bacterial extracts were tested also for their putative antagonistic or additive properties versus Pam3Cys added just after the extracts.

Bacterial extract OP0701 B4 AFer50

Table 4 IL-8 (pg/ml) secretion from supernatants obtained from HEK TLR2/6 cells stimulated at the indicated concentrations with the controls (medium, LPS, or Pam3Cys), with the bacterial extract OP0701 B4_AFer50 or with the extract and Pam3Cys.

| Pam3Cys | | 0 0003 μg/ml | 0 001 μg/ml | 0 003 μg/ml | 0 01 μg/ml |
|---|---|---|---|---|---|

(continued)

| | | 18 | 88 | 269 | 696 |
|---|---|---|---|---|---|
| | | | | | |
| Medium | n a | 2 pg/ml IL-8 | | | |
| LPS E coli K12 | 0 01 μg/ml | 2 pg/ml IL-8 | | | |
| | | | | | |
| OP0701 B4_Afer50 | pg/ml IL-8 | Bacterial Extract + **Pam3Cys 0.0003 μg/ml** pg/ml IL-8 | Bacterial Extract + **Pam3Cys 0.001 μg/ml** pg/ml IL-8 | Bacterial Extract + **Pam3Cys 0.003 μg/ml** pg/ml IL-8 | Bacterial Extract **+Pam3Cys 0.01 μg/ml** pg/ml IL-8 |
| 0 2 mg/ml | 132 | 180 | 314 | 566 | 1095 |
| 0 5 mg/ml | 462 | 546 | 713 | 900 | 1742 |
| 1 mg/ml | 1218 | 1243 | 1307 | 1980 | 2297 |
| 2 mg/ml | 1830 | 2314 | 2595 | 2639 | 2683 |

The results in Table 4 indicate that the OP0701 B4_AFer50 extract induced the production of high levels of IL-8 (agonist TLR 2/6).

Bacterial extract OP0701 C-Bt1 LAC

Table 5:IL-8 (pg/ml) secretion from supernatants obtained on HEK TLR2/6 cells stimulated at the indicated concentrations with the controls (medium, LPS, or Pam3Cys), with the bacterial extract OP0701 C-Bt1 LAC or with the extract and Pam3Cys.

| Pam3Cys | | 0.0003 μg/ml | 0.001 μg/ml | 0.003 μg/ml | 0.01 μg/ml |
|---|---|---|---|---|---|
| | | 18 | 88 | 269 | 696 |
| | | | | | |
| Medium | n.a | 2 pg/ml IL-8 | | | |
| LPS E coli K12 | 0.01 μg/ml | 2 pg/ml IL-8 | | | |
| | | | | | |
| OP0701C-Bt1 LAC | pg/ml IL-8 | Bacterial Extract + **Pam3Cys 0.0003 μg/ml** pg/ml IL-8 | Bacterial Extract + **Pam3Cys 0.001 μg/ml** pg/ml IL-8 | Bacterial Extract + **Pam3Cys 0.003 μg/ml** pg/ml IL-8 | Bacterial Extract **+Pam3Cys 0.01 μg/ml** pg/ml IL-8 |
| 0.2 mg/ml | 347 | 499 | 488 | 824 | 1311 |
| 0.5 mg/ml | 1504 | 1447 | 1577 | 1889 | 2213 |
| 1 mg/ml | 2740 | 2966 | 2295 | 3023 | 2649 |
| 2 mg/ml | 4306 | 4318 | 3744 | 3540 | 3454 |

The results in Table 5 indicate that the OP0701 C-Bt1 LAC extract induced the production of high levels of IL-8 (agonist TLR 2/6).

Bacterial extract OP0701 C-Bt2LAC

Table 6:IL-8 (pg/ml) secretion from supernatants obtained on HEK TLR2/6 cells stimulated at the indicated concentrations with the controls (medium, LPS, or Pam3Cys), with the bacterial extract OP0701 C-Bt2LAC or with the extract and Pam3Cys.

| Pam3Cys | | 0.0003 µg/ml | 0.001 µg/ml | 0.003 µg/ml | 0.01 µg/ml |
|---|---|---|---|---|---|
| | | **18** | **88** | **269** | **696** |
| | | | | | |
| Medium | n.a | 2 pg/ml IL-8 | | | |
| LPS E coli K12 | 0.01 µg/ml | 2 pg/ml IL-8 | | | |
| | | | | | |
| OP0701C-Bt2LAC | pg/ml IL-8 | Bacterial Extract + **Pam3Cys 0.0003 µg/ml** pg/ml IL-8 | Bacterial Extract + **Pam3Cys 0.001 µg/ml** pg/ml IL-8 | Bacterial Extract + **Pam3Cys 0.003 µg/ml** pg/ml IL-8 | Bacterial Extract **+Pam3Cys 0.01 µg/ml** pg/ml IL-8 |
| 0.2 mg/ml | 9 | 17 | 60 | 209 | 571 |
| 0.5 mg/ml | 8 | 12 | 41 | 175 | 448 |
| 1 mg/ml | 11 | 18 | 36 | 120 | 361 |
| 2 mg/ml | 13 | 15 | 24 | 59 | 206 |

The results in Table 6 indicate that the OP0701 C-Bt2LAC extract did not induce the production of IL -8 (agonist TLR 2/6), and in the presence of Pam3Cys had an antagonist effect on TLR2/6.

Conclusion

Depending on the conditions of the alkaline lysis (initial concentration of biomass dry weight, initial base concentration and duration of base treatment), the bacterial extracts could have different modes of action.

OP0701 B4_Afer50 and OP0701 C-Bt1 LAC were agonist TLR2/6 but the stronger alkaline lysis performed on the same bacterial strain resulted in an antagonistic TLR2/6 activity (OP0701 C-Bt2LAC).

b) HEK-TLR4-MD2-CD14

TLR4 was extensively studied since it is the major receptor involved in the recognition of lipopolysaccharide (LPS) responsible for sceptic shock.

HEK-TLR4-MD2 -CD14 cells are highly sensitive to LPS. They were obtained by stable transfection of HEK293 cells with the TLR4, MD2 and CD14 genes and an NF-κB-inducible reporter system. These cells secrete IL-8.

The same experimental procedure was employed as for the other HEK cell line TLR2/6 described above. The results for the controls and the 3 bacterial extracts of the invention tested are shown in Tables 7-9..

Results: IL-8 secretion

The results for the controls (positive = LPS K12C ultrapure, TLR4 agonist; and PAM3CSK4 = negative, TLR2 agonist) are provided in Tables 7-9. The results (expressed as pg/ml of IL-8) show the mean values of IL-8 secretion 24 hours after stimulation with the controls.

The cell line responds clearly only to the TLR4 agonist LPS K12. In contrast, as expected, the TLR2 agonist from Pam3Cys was inactive, even at the high dose of 0.01 µg/ml.

Experiments with the 3 bacterial extracts alone

As expected from Gram positive bacteria agonists, the 3 bacterial extracts tested here (OP0701 B4 Afer50: Table 7, OP0701 C-Bt1 LAC: Table 8, and OP0701 C-Bt2LAC: Table 9) exhibited no clear immunostimulating properties via the TLR4 pathway.

Experiments with the 3 bacterial extracts + LPS K12

As mentioned above, the three extracts of the invention were tested also for their putative antagonistic or additive properties versus LPS added just after the extracts. Again, no effect was observed at the level of the TLR4 receptor (Tables 7-9).

Bacterial extract OP0701 B4 AFer50

Table 7: IL-8 (pg/ml) secretion from supernatants obtained on HEK TLR4 cells stimulated at the indicated concentrations with the controls (medium, LPS, or Pam3Cys), with the bacterial extracts OP0701 C-AFer50 or with the extracts and LPS K12.

| LPS K12 | | 0.0003 µg/ml | 0.001 µg/ml | 0.003 µg/ml | 0.01 µg/ml |
|---|---|---|---|---|---|
| | | 251 | 477 | 802 | 1383 |

| Medium | n.a | 0 pg/ml IL-8 |
|---|---|---|
| Pam3Cys | 0.01 µg/ml | 138 pg/ml IL-8 |

| | pg/ml I L-8 | Bacterial Extract **+ LPS K12 0.0003 µg/ml** pg/ml IL-8 | Bacterial Extract **+ LPS K12 0.001 µg/ml** pg/ml IL-8 | Bacterial Extract **+ LPS K12 0.003 µg/ml** pg/ml IL-8 | Bacterial Extract **+LPS K12 0.01 µg/ml** pg/ml IL-8 |
|---|---|---|---|---|---|
| OP0701B4_Afer50 | | | | | |
| 0.2 mg/ml | 152 | 1 | 502 | 856 | 1446 |
| 0.5 mg/ml | 176 | 335 | 458 | 736 | 1511 |
| 1 mg/ml | 192 | 391 | 529 | 956 | 1580 |
| 2 mg/ml | 198 | 422 | 736 | 935 | 1553 |

The results indicate that the OP0701 B4_AFer50 extract did not activate TLR4 receptor (Pam3Cys: 138 pg/mL).

Bacterial extract OP0701 CBt1-LAC

Table 8: IL-8 (pg/ml) secretion from supernatants obtained on HEK TLR4 cells stimulated at the indicated concentrations with the controls (medium, LPS, or Pam3Cys), with the bacterial extracts OP0701 C-Bt1 LAC or with the extracts and LPS K12.

| LPS K12 | | 0.0003 µg/ml | 0.001 µg/ml | 0.003 µg/ml | 0.01 µg/ml |
|---|---|---|---|---|---|
| | | 251 | 477 | 802 | 1383 |

| Medium | n.a | 0 pg/ml IL-8 |
|---|---|---|
| Pam3Cys | 0.01 µg/ml | 138 pg/ml IL-8 |

(continued)

| OP0701C-Bt1LAC | pg/ml IL-8 | Bacterial Extract **+ LPS K12 0.0003** **µg/ml** pg/ml IL-8 | Bacterial Extract **+ LPS K12 0.001** **µg/ml** pg/ml IL-8 | Bacterial Extract **+ LPS K12 0.003** **µg/ml** pg/ml IL-8 | Bacterial Extract **+LPS K12 0.01** **µg/ml** pg/ml IL-8 |
|---|---|---|---|---|---|
| 0.2 mg/ml | 140 | 282 | 471 | 795 | 1345 |
| 0.5 mg/ml | 152 | 292 | 502 | 778 | 1537 |
| 1 mg/ml | 152 | 318 | 616 | 952 | 1576 |
| 2 mg/ml | 162 | 322 | 632 | 1144 | 1716 |

Bacterial extract OP0701 CBt2-LAC

Table 9 :IL-8 (pg/ml) secretion from supernatants obtained on HEK TLR4 cells stimulated at the indicated concentrations with the controls (medium, LPS, or Pam3Cys), with the bacterial extracts OP0701C-Bt2LAC or with the extracts and LPS K12.

| LPS K12 | | 0.0003 µg/ml | 0.001 µg/ml | 0.003 µg/ml | 0.01 µg/ml |
|---|---|---|---|---|---|
| | | 251 | 477 | 802 | 1383 |

| Medium | n.a | 0 pg/ml IL-8 |
|---|---|---|
| Pam3Cys | 0.01 µg/ml | 138 pg/ml IL-8 |

| OP0701C-Bt2LAC | pg/ml IL-8 | Bacterial Extract **+ LPS K12 0.0003** **µg/ml** pg/ml IL-8 | Bacterial Extract **+ LPS K12 0.001** **µg/ml** pg/ml IL-8 | Bacterial Extract **+ LPS K12 0.003** **µg/ml** pg/ml IL-8 | Bacterial Extract **+LPS K12 0.01** **µg/ml** pg/ml IL-8 |
|---|---|---|---|---|---|
| 0.2 mg/ml | 148 | 213 | 449 | 713 | 1111 |
| 0.5 mg/ml | 154 | 225 | 333 | 622 | 1228 |
| 1 mg/ml | 125 | 219 | 375 | 782 | 1306 |
| 2 mg/ml | 105 | 259 | 391 | 799 | 1164 |

Conclusion

Taken together, the results presented here on HEK cells, and those obtained on human PBMC cells, suggest that Afer50 and BT1 LAC are able to stimulate the immune system via the TLR2 pathway. Moreover, BT2LAC may behave as a TLR2/6 antagonist. Hence, extracts according to the invention may stimulate the immune system via the TLR2 pathway or act as TLR2/6 antagonists, thus correlating to anti-infection and anti-inflammatory activities *in vivo.*

**Example 8:**

Cells that express a given functional TLR gene are valuable tools for many applications, such as the study of the mechanisms involved in TLR recognition or signaling, and the development of new potential therapeutic drugs. It was therefore the aim of the experiments described below to test the activity of 4 bacterial extracts on these key adaptors of the immune response.

In this test, 8 TLR and NOD2 receptors were screened for 4 bacterial extracts, including extracts according to the present invention.

Method

Bacterial extracts according to the invention were tested in 96 well microplates. The extracts were diluted in DMEM culture medium and 20 μl of each dilution was tested in duplicate. A volume of 180 μl of HEK293 cell suspensions containing 25000 or 50000 cells in DMEM culture medium + 10% FCS (one HEK293 cell line specific for each TLR with reporter gene secreted alkaline phosphatase under the control of NF-kB) were added into each well in duplicate. After 16 hours incubation with each cell line, 20 to 50 μl of each supernatant was transferred into 96 well microplates and completed with 200 μl of Quantiblue (InVivoGen no REP-QB1). Enzymatic reaction with secreted alkaline phosphatase was performed for 30 to 60 min for the different series of TLRs expressing cell lines. Read out was performed using a microplatereader at 630 nm. Results are expressed in OD at 630 nm.

Material

List of positive control agonists (and their respective concentrations) used in this screening assay

TLR2 PAM2 100 ng/ml; TLR3 Poly(I:C) 100 ng/ml; TLR4 *E. coli K12* LPS 1 μg/ml; TLR5 S. typhimurium flagellin 1 μg/ml; TLR7 R848 10 μg/ml; TLR8 R848 10 μg/ml; TLR9 CpG ODN 2006 10 μg/ml; NOD2 Muramyldipeptide 1 μg/ml.

Negative controls

A recombinant HEK-293 cell line for the reporter gene only (NFkB) was used as a negative control for the TLR cell lines. The negative control value for each clone was the background signal of these non-induced clones. TNF-alpha was used as a positive control for this non-TLR expressing cell line.

Test articles

The following bacterial extracts were tested:
OP0701 B4_CFer300: extract from *Lactobacillus fermentum 1-3929* (12 g/L) obtained as described in Example 3.3 (F);
OP0701 B4_CRahr300: extract from *Lactobacillus rhamnosus 71.38* (40 g/L) obtained as described in Example 3.4 (G);
OP0701 D_10L1 PswitchA: extract from *Lactobacillus fermentum I-3929* (10 g/L) obtained as described in Example 3.12 (I); and
OP0701 D_5LOSMOConc: extract from osmotic lysis *Lactobacillus fermentum I-3929* (7.6 g/L) obtained by osmotic stress in 0.16 N NaCl solution at 40 °C for 24 hours (H) as a control.

Preparation of bacterial extracts

20 μl of each sample to be tested was used to stimulate all the cell lines in a 200 μL reaction volume.
Screening was performed at a single concentration, typically a 0.5 mg of dry weight /mL. The tests were performed in duplicate.

Results

The bacterial extracts and controls were tested in duplicate on recombinant HEK-293 cell line that functionally express a given TLR or NOD2 protein as well as a reporter gene driven by NFkB promoter. TLR and NOD2 activation results are given as optical density (OD) values. The results are shown in Tables 10-14, and summarized in Table 15.

Bacterial extract F (OP0701 B4Cfer300)

Table 10: activation of TLR's and NOD2 receptors by OP0701 B4Cfer300 extracts.

|  | hTLR2 | hTLR3 | hTLR4 | hTLR5 | hTLR7 | hTLR8 | hTLR9 | hNOD-2 |
|---|---|---|---|---|---|---|---|---|
| Reference | 3.493 | 2.052 | 1.341 | 3.657 | 1.480 | 2.786 | 1.955 | 0.576 |
| Extract F | 3.963 | 0.051 | 0.735 | 0.304 | 0.070 | 0.080 | 0.026 | 0.503 |

Bacterial extract F activated specifically hTLR2, hTLR4 and hNod2, and to a lesser extent, hTLR5.

Bacterial extract G (OP0701 B4CRahr300)

Table 11: activation of TLR's and NOD2 receptors by OP0701 B4CRahr300 extracts.

|  | hTLR2 | hTLR3 | hTLR4 | hTLR5 | hTLR7 | hTLR8 | hTLR9 | hNOD-2 |
|---|---|---|---|---|---|---|---|---|
| Reference | 3.493 | 2.052 | 1.341 | 3.657 | 1.480 | 2.786 | 1.955 | 0.576 |
| Extract G | 0.808 | 0.003 | 0.177 | 0.017 | 0.028 | 0.044 | -0.032 | 0.369 |

Bacterial extract G activated hTLR2, hNOD2, and to a much lesser extent, hTLR4.

Bacterial extract H (OP0701D 5LOSMOConc)

Table 12: activation of TLR's and NOD2 receptors by OP0701 D_5LOSMOConc extracts.

|  | hTLR2 | hTLR3 | hTLR4 | hTLR5 | hTLR7 | hTLR8 | hTLR9 | hNOD-2 |
|---|---|---|---|---|---|---|---|---|
| Reference | 3.493 | 2.052 | 1.341 | 3.657 | 1.480 | 2.786 | 1.955 | 0.576 |
| Extract H | 4.537 | 0.057 | 0.638 | 0.050 | -0.072 | 0.054 | 0.011 | 0.892 |

Bacterial extract H specifically activated hTLR2, hTLR4 and Nod2.

Bacterial extract I (OP0701D 10L1 PswitchA)

Table 13 : activation of TLR's and NOD2 receptors by OP0701 D_10LPswitchA extracts.

|  | hTLR2 | hTLR3 | hTLR4 | hTLR5 | hTLR7 | hTLR8 | hTLR9 | hNOD-2 |
|---|---|---|---|---|---|---|---|---|
| Reference | 3.493 | 2.052 | 1.341 | 3.657 | 1.480 | 2.786 | 1.955 | 0.576 |
| Extract I | 4.168 | 0.096 | 0.342 | 3.490 | -0.024 | 0.132 | 0.410 | 0.790 |

Bacterial extract I strongly activates hTLR2, hTLR5, hNOD2, and to a lesser extent, hTLR4 and hTLR9.

Table 15: activation of TLR's and NOD2 receptors by different bacterial extracts obtained from different process conditions according to the present invention.

|  | hTLR2 | hTLR3 | hTLR4 | hTLR5 | hTLR7 | hTLR8 | hTLR9 | hNOD-2 |
|---|---|---|---|---|---|---|---|---|
| Extract F | + | - | + | +/- | - | - | - | + |
| Extract G | + | - | +/- | - | - | - | - | + |
| Extract H | + | - | + | - | - | - | - | + |
| Extract I | + | - | +/- | + | - | - | +/- | + |

Thus the bacterial extracts according to the present invention may help to activate the immune system via various TLRs, and also Nod2. Therefore the extracts of the invention may be good activators of immune response.

Comparing extract H to extract G and extract F, it becomes apparent that the extracts according to the invention have the same TLR's and Nod pathway as extracts obtained from an osmotic lysis. Comparing extract H and extract I, we may observe that the TLR5 pathway was activated, and the TLR9 pathway to a lesser extent, by the addition of an acid lysis following the alkaline one. Even extract F, resulting from a weak alkaline process, showed activation of the TLR5 pathway as opposed to extract H.

Extract I acted on TLR5, which indicates a potential application in radioatherapy. Indeed, radiation therapy is a well-established and highly effective treatment for certain types of cancer. Its side effects can be devastating, however, as it can destroy healthy cells in the body, especially bone-marrow cells and cells in the gastrointestinal tract. Burdelya et al. reported recently that a CBLB502 peptide binds to TLR5 and activates the nuclear factor-κB signaling pathway, a pathway that cancer cells often activate to avoid cell death (Burdelya et al., "An agonist of toll-like receptor 5 has radioprotective activity in mouse and primate models," Science, 2008, 320:226-30). Mice and rhesus monkeys treated with CBLB502 shortly before exposure to lethal doses of total body irradiation exhibited less damage to healthy bone marrow and gastrointestinal cells, and survived significantly longer than controls. Importantly, in tumor-bearing mice, CBLB502 did not compromise the antitumor efficacy of radiation therapy.

## Example 9: Ability of extract to produce plaque forming cells (against sheep red blood cells) in mice

The plaque forming cells (PFC) technique enables the evaluation of a non-specific stimulation of B-lymphocytes. This technique was first described by Cunningham and Seenberg (Immunology, 1968, 14, 599). The presence of hemolytic antibodies around antibody-forming cells was demonstrated as described below. Murine lymphoid cells, as well as a dense population of foreign (sheep) erythrocytes, were simultaneously introduced on a microscope slide. Certain lymphoid cells release hemolytic antibodies which diffuse and cause the lysis of the neighboring red blood cells by forming a lysis plaque in the presence of complement. At the end of the experiment, the number of PFC reported to $10^6$ cells or to the spleen was counted.

### Materials and Methods

### Test articles

Bacterial extract 1 obtained as described in Example 3.5, and bacterial extract 2 obtained as described in Example 3.4.

### Animals

40 male Balb/C mice/experiment (IFFA-CREDO, St. Germain sur l'Arbresle Cedex, France), 5 to 6 weeks old and with a mean weight of 20 +/- 2 g were distributed in 5 groups of 8 animals each.

### Experimental design

Animals were divided into 5 groups as follows:

Group (a) 8 mice receiving 1.2 mg/mouse of extract 1 obtained as described in Example 3.5; volume = 0.2 ml

Group (b) 8 mice receiving 0.6 mg/mouse of extract 1 obtained as described in Example 3.5.

Group (c) 8 mice receiving 1.2 mg/mouse of extract 2 obtained as described in Example 3.4; volume = 0.2 ml

Group (d) 8 mice receiving 0.6 mg/mouse of extract 2 obtained as described in Example 3.4; volume = 0.2 ml

Group (e) 8 mice receiving 0.2 ml isotonic saline

Mice received the extracts described above, at the doses indicated, daily per os (via the oral route) for 5 consecutive days (day 1 to day 5). Two more intubations occurred on days 19 and 20. On day 29, the antigen ($10^6$ sheep red blood cells) in 0.2 ml isotonic saline (0.9% NaCl, Alsever solution) was injected invtraveneously through the caudal vein of the animal; 4 days later (day 33) the spleen cell suspensions were prepared.

Reagents and equipment

Alsever's solution, endotoxin free, (Sigma, 38299 St Quentin Fallavier, Cedex France, ref. A 3351)

Basal Medium Eagle (BME, 10x concentrated) in bicarbonate-free Earle's solution (Bio-Mérieux, 69280 Marcy l'Etoile, France ref: 8 210 2)

Lyophilized guinea pig serum (Bio-Mérieux, Marcy l'Etoile, France ref : 7 212 2)

Sheep red blood cells (Bio-Mérieux, Marcy l'Etoile, France ref: 72141)

Trypan blue, sterile distilled water, NaCl 0.9%, tissue homogenizer, centrifuge, Neubauer chamber for the numeration of cells, glass tubes.

Treatment with test or control article

Control animals received Sheep Red Blood Cells (SRBC) only. Animals of other groups received the bacterial extracts *per os* as indicated previously. To this end, each extract was dissolved in water.

Spleen cells suspension

4 days after the last antigen (SRBC) injection, a suspension of spleen cells was prepared for each mouse according to the following procedure:

The animal was anesthetized with ether and then sacrificed by cervical dislocation. The spleen was removed and crushed in a glass tissue homogenizer with 2 ml BME at pH 6.75-6.80. Then 3 ml of sterile distilled water were added, and the mixture was stirred for 20 seconds. The suspension obtained was further diluted with 10 BME and centrifuged at 1200 rpm for 10 minutes at 4°C. The supernatant was discarded and the precipitate was suspended in 2 ml BME. The cells were allowed to recover 5-10 minutes on ice. A viability count of the mononucleated cells was performed in the following way:

A 1/20 dilution of an aliquot of the cell suspension was prepared with saline containing a 0.1 % solution of trypan blue. The non-colored cells, i.e. the living cells, were counted in a Neubauer chamber.

The cell suspension was kept in melting ice during the counting and then immediately used for the lysis plaques. This technique prevents a loss in viability which may happen if the wait is too long.

Preparation of slides

On an ordinary microscope slide, carefully dusted and with any fat trace removed, 3 parallel strips (0.1 mm thick) of double-sided tape were stuck at intervals of about 1.5 cm. The strips were then covered with pre-cleaned coverslips (22 mm x 22 mm) to form two "chambers" between the slide and the coverslips.

A fraction of the spleen cell suspension was adjusted to 25,000 mononucleated cells per mm$^3$. In a small glass tube, a mixture of the following elements (added in the order described) was prepared by means of an automatic pipette:

1) 7.5 ml BME

2) 0.5 ml of the SRBC suspension washed and centrifuged twice with saline, at 50 % residue

3) 0.4 ml of normal guinea pig serum used as source of complement

Finally, to 50 $\mu$l of the spleen cell suspension at 25,000 cells/mm$^3$ was added to 200 $\mu$l of the preceding mixture. After a soft homogenization, the suspension was introduced in "Cunningham Chambers" by capillary action before sealing the free sides with paraffin. The slides were then placed in a humid chamber in an oven at 37°C.

Lecture

After one hour incubation in the oven the slides were examined under microscope enlarged 100 times (ocular 10 X, objective 10 X).

The lysis plaques were easy to recognize. 5 vertical optic strips were examined on each slide and the number of lysis plaques was counted. The number of cells (N) forming direct lysis plaques per $10^6$ cells was calculated by extrapolation. If x is the number of observed plaques and X the number of examined cells, the number of direct lysis plaques per $10^6$ cells is equal to:

$$N = (x / X) * 10^6$$

where X=C*V=C*(n*e*I*L)
C = final concentration of spleen cells
V = observed volume
n = number of optic strips
e = thickness of the adhesive tape
I = width of the optic field
L = length of an optic strip

The number of direct lysis plaques per $10^6$ cells was obtained from the following equation:

$$PFC \ (per \ 10^6 \ cells) = (x * 10^6) / (C * n * e * I * L)$$

By knowing the number of cells collected per spleen, it was possible to deduce the number of cells forming lysis plaques per spleen.

In the "absolute reference" mice, no spontaneous lysis plaque was observed.

Statistical analysis and Results

Results were considered significant when $p < 0.05$ (Students t test). The results are shown in Table 16.

Table 16: Ability of the bacterial extracts 1 and 2 to produce plaque forming dells (against sheep red blood cells) in mice

|  | GRM | PFC/$10^6$ cellules | PFC/rate |
|---|---|---|---|
| Extract 1; see Example 3.5 (1.2 mg/mouse/administration) | 100%<br>100%<br>100% | 124%*<br>123%*<br>114%* | 119%*<br>143%*<br>113% |
| Extract 1; see Example 3.5 (0.6 mg/mouse/administration) | 100%<br>100%<br>100% | 117%*<br>118%*<br>112%* | 103%<br>139%*<br>128%* |
| Extract 2; see Example 3.4 (1.2 mg/mouse/administration) | 100%<br>100%<br>100% | 119%*<br>124%*<br>117%* | 109%<br>131%*<br>120%* |
| Extract 2; see Example 3.4 (0.6 mg/mouse/administration) | 100%<br>100%<br>100% | 117%*<br>114%*<br>113%* | 112%*<br>119%*<br>111% |

The extracts of the invention appeared to be B-cell activators when tested at concentrations of 1.2 mg/mouse/per day or 0.6 mg/mouse/day. A slight dose-dependent effect was observed.Hence, some extracts of the invention could potentially be used to prime the immune system in patients suffering from recurrent infections.

**Example 10: Effect of CFer300 in intraperitoneal Salmonella typhimurium infection in balb/c mice**

Protection of mice infected with *Salmonella typhimurium* was tested by bacterial lysates from *Lactobacillus* origin after oral administration.

Materials and Methods

Animals and Husbandry

Balb/c mice were housed in the facilities of the Institute for Immunology, Moscow. For the *in vivo* protection experiment, non-inbred laboratory grade white mice were purchased from Stolbovaya, the Russian State Scientific Centre for Biomedical Technologies (Russian Academy of Medical Sciences). Upon arrival, the mice had a body weight of 12-14 g. Throughout the experiments, mice were maintained at pathogen-free conditions on standard rodent diet and water.

Study groups (main experiment)

Two groups of 22 mice per group were used to test the anti-infective efficacy of the bacterial extracts prepared using an experimental model of *Salmonella typhimurium* infection in mice.

One group was treated orally with a solution of CFer300, and the second group received a sham treatment (water) as a negative control.

0.5 ml of the solution was given to each mouse orally once a day for 10 consecutive days before all mice were challenged with *Salmonella typhimurium:*

Group 1: Mice treated with CFer300 given per os as single 2 mg (0.5 ml) doses.

Group 2: Mice received a sham treatment using oral administration of 0.5 ml water daily for 10 days.

Test article

The bacterial extract tested was OP0701 B4CFer300 ("CFer300"); the extract from *Lactobacillus fermentum* I-3929 (12 g/L) obtained as described in Example 3.3 (28.4 mg of active dry weight /g).

Preliminary experiment

The purpose of the preliminary experiment was to determine the dose of the infectious agent that would induce mortality close to 50% three weeks after the challenge. As challenge, a suspension of *Salmonella enterica, serovar typhimurium strain* 415 (I. Mechnikov, Institute for Vaccines and Sera, Russian Academy of Medical Sciences) was injected intraperitoneally into each mouse. The challenge dose ranged from $10^3$ to $10^5$ CFU of *Salmonellae* per mouse.

Observations and death record

After the challenge, mice were kept under standard conditions for laboratory animals. Daily observations and records of death were recorded during a period of 21 days post infection. The anti-infective efficacy of the preparations was estimated according to the post infection survival rate (SR), the post infection average duration of life (ADL), the defense factor (DF), and the preparation efficacy index (EI), calculated for each experimental group. The SR was taken as percent of animals alive in the experimental groups on day 21 post infection. The ADL, DF, and EI were calculated as follows:

$$ADL = (X1 + X2 + \ldots + Xn) / N$$

where:
ADL is the average duration of life,
X1 to Xn are the durations of life post-infection for experimental mice 1 to n, and N is the total number of animals in the experimental group.

$$DF = CD / ED$$

where:
DF is the defense factor,

CD is the percent of death in the control group, and
ED is the percent of death in the experimental group.

$$EI = [(DF - 1) / DF] \times 100\%$$

where:
EI is the preparation efficacy index, and
DF is the defense factor.

Results: drug tolerance

The preparation was given orally once a day for 10 days and was tolerated well. Evidences of of toxicity or side effects were not observed during the 10 days period of pretreatment.

Titration of *Salmonellae*

A preliminary experiment was performed in order to determine the challenge dose of *Salmonella typhimurium* to results in approximately 50% death rate. The results are shown in Table 17.

Table 17: Preliminary experiment to determine the challenge dose of *Salmonella typhimurium* corresponding to approximately 50% death rate.

| S.typhi dose | Mice/ group | Days post-infection | | | | | | | | | | | | | Dead mice / total | Dead (%) |
|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | | |
| **$10^5$** | **6** | - | - | - | - | <u>1</u> | <u>2</u> | - | - | - | - | <u>1</u> | - | - | **4/6** | **67** |
| $10^4$ | 6 | - | - | - | - | - | - | - | <u>1</u> | - | - | - | - | - | 1/6 | 17 |
| $10^3$ | 6 | - | - | - | - | - | - | - | - | - | - | - | - | - | 0/6 | 0 |

The underlined numbers represent the number of animals found dead on the day (post infection) indicated.

Conclusion

On the basis of the data obtained, the dose of $10^5$ CFU of *Salmonella typhimurium* strain 415 (67% of dead animals, in bold) was chosen for the subsequent study.

Main experiment: Challenge of mice treated with CFer300

Mice pretreated for 10 days with the CFer300 preparation (n=22) or, in the control group with water (n=22) were challenged the day after the end of pretreatment.
A suspension of *Salmonella typhimurium* was administered intraperitoneally as a $10^5$ CFU challenge dose per mouse. A follow-up observation was performed 21 days post-infection. The death rate of the groups is shown in Table 18.

Table 18: Follow up observation 21 days post-infection

| Drug | Days post-infection | | | | | | | | | | | | | | | | | | | | | Death (%) | Survival Rate (%) |
|---|---|---|---|---|---|---|---|---|---|---|---|---|---|---|---|---|---|---|---|---|---|---|---|
| | 1 | 2 | 3 | 4 | 5 | 6 | 7 | 8 | 9 | 10 | 11 | 12 | 13 | 14 | 15 | 16 | 17 | 18 | 19 | 20 | 21 | | |
| CFer 300 | - | - | - | - | *1* | *2* | - | *1* | - | *1* | *1* | - | - | - | - | - | - | - | - | - | - | 27 | 73 |
| $H_2O$ | - | - | - | *1* | *2* | *2* | *2* | - | - | - | - | *1* | - | - | *1* | *1* | - | - | - | - | - | 45 | 55 |

The numbers *in italic* represent the number of animals found dead on the day (post infection) indicated. These data were used to calculate SR, ADL, DF and EI (Table 19).

Table 19: survival rate during the period of observation (21 day)

| Pre-Treatment With Substances | Death Rate (%) | Survival Rate (%) | ADL (days) | DF | EI (%) |
|---|---|---|---|---|---|
| **Cfer 300** | 27 | 73 | 17.1 | 1.67 | 40% |
| $H_2O$ | 45 | 55 | 14.7 | 1 | 0 |

In the control group pretreated with water, a survival rate during the period of observation (21 day) was 55%, and the ADL was 14.7 days. At the dose of 2 mg/day, CFer300 exhibited a protective effect, resulting in SR=73% and ADL=17.1 days, i.e., DF = 1.67 and EI = 40%.

In summary, a 10 day course of a daily oral treatment with CFer300 given in single doses of 2 mg offered partial protection in mice infected by *Salmonella typhimurium.* As shown in this example, and shown previously *in vitro* and *ex-vivo,* the extracts presently disclosed may be useful in further therapeutic drug development.

### Example 11: Effect of two *Lactobacillus* extracts in a model of LACK-induced asthma

Two embodiments of the invention were tested in a murine model of allergen-induced asthma after oral administration (Julia et al., Immunity, 2002, 16:271-283).

Materials and Methods

Animals and Husbandry

6 weeks old female BALB/c ByJ mice were purchased from Janvier, France. They were maintained and fed under standard conditions.

Study Groups

A total of 27 mice were divided into 4 groups as follows:

Group A: untreated, LACK-sensitized and saline-challenged mice; LACK is a protein from the parasite Leishmania major (4 mice).

Group B: untreated LACK-sensitized and challenged mice (8 mice)

Group C: OM-1 009A-treated (8 mg of dry weight residue per administration), LACK-sensitized and challenged mice (8 mice)

Group D: OM-1009B-treated (8 mg of dry weight residue per administration), LACK-sensitized and challenged mice (7 mice)

Treatment and schedule with test or control article

Mice were treated from day -3 to day 22. On day 0 and day 7, mice were sensitized intraperitoneally (i.p.) with 10 $\mu$g of LACK in the presence of 2 mg of alum 3. From day 17-21, mice were exposed to a daily 20-min aerosol challenge of a LACK solution (0.15%) (Groups B, C, and D), or a saline solution (Group A) as control. On day 22, mice were analyzed for their ability to develop AHR upon methacholine inhalation. On day 23, the mice were sacrificed and lung inflammation was assessed.

Methodology

Mice were therapeutically treated three times as described above. Lavages were performed in individual mice bled with a canula inserted into their trachea. Lungs were washed 3 times with 1 ml of warmed PBS. Cells were washed with PBS, resuspended in 300 $\mu$l, and counted using a Burker-Turk chamber. For differential BAL cell counts, cytospin preparations were made and stained with Wright/Giemsa coloration. The groups tested were: LACK-sensitized and PBS-challenged wild type (wt) mice (Control), LACK-sensitized and challenged wt mice (asthmatic animals), OM-1 009A-treated wt mice, and OM-1009B-treated wt mice. The total cell number in BAL was determined by microscopic examination of cytospin preparations stained with Wright/Giemsa coloration.

As a decrease in airway inflammation was observed in the treated mice (see results below), lung extracts were prepared, and IL-4, IL-5, and IL-13 were quantified by multiplex analysis (CBA array).

Test articles

The two bacterial lysates tested were: OM-1 009A extract from *Lactobacillus fermentum* I-3929 obtained as described in Example 3.7 and OM-1009B extract from *Lactobacillus fermentum* I-3929 obtained as described in Example 3.11.

Results

a) Airway hyperresponsiveness

On day 22, mice were analyzed for their ability to develop AHR upon methacholine inhalation at the doses indicated. The results are reported in Figure 5. The results show that OM-1 009-B restored basal Penh values similar to the PBS untreated non-asthmatic group. OM-1 009-A was more efficient, since the Penh values obtained were even lower than those observed in the non-asthmatic control group.

b) Total and differential cell number in broncho-alveolar lavages

On day 23, mice were sacrificed and lung inflammation was assessed. The total cell number in BAL is reported in Table 20 (right column), and also the differential cell numbers: Eosinophils number (Eo), Neutrophils number (Neutro), Lymphocytes number (Lympho), and Other cells number (Other).

Table 20: Total and differential cell number in BAL upon treatment with two extracts of the invention (Groups C and D). Mean values and standard error mean values are provided.

| Mean | Eo | Neutro | Lympho | Others | total cells |
|---|---|---|---|---|---|
| (A) Ctrl PBS | 901 | 3178 | 11796 | 49125 | 65000 |
| (B) Ctrl LACK = asthmatic mice | 347691 | 51317 | 85609 | 301097 | 785714 |
| (C) OM-1009A+ LACK | 115227 | 27879 | 54725 | 158419 | 356250 |
| (D) OM-1009B+ LACK | 203834 | 14210 | 34523 | 204575 | 457143 |

| SEM | Eo | Neutro | Lympho | Others | total cells |
|---|---|---|---|---|---|
| (A) Ctrl PBS | 166 | 906 | 6255 | 5568 | 2887 |
| (B) Ctrl LACK | 78464 | 14392 | 17736 | 78575 | 135275 |
| (C) OM-1009A+ LACK | 36741 | 10444 | 27024 | 34296 | 94905 |
| (D) OM-1009B+ LACK | 57955 | 3220 | 6731 | 25311 | 74992 |

Both extracts decreased significantly the total cell number in BAL (p<0.01 for OM-1 009A; and p<0.03 for OM-1009B when compared to group B). The eosinophils numbers were decreased 3- and 1.7-fold respectively. The neutrophils numbers were decreased 1.8- and 3.6-fold respectively. The reduction of the eosinophil and neutrophil numbers indicates a lower concentration of inflammatory cells in BAL of animals pretreated with the extracts.

c) Th2 cytokines detected in lung extracts and quantified by multiplex anal, sis (CBA array)

Table 21 individual IL4 lung levels in mice.

| pg/mg | mouse 1 | mouse 2 | mouse 3 | mouse 4 | mouse 5 | mouse 6 | mouse 7 | mouse 8 | Mean |
|---|---|---|---|---|---|---|---|---|---|
| (A) PBS | 0 | 0.0053 | 0.0071 | 0.0157 | | | | | **0.0070** |
| (B) LACK (asthmatic mice) | 0.0395 | 0.0141 | 0.0279 | 0.0203 | 0.0353 | 0.0574 | 0.0949 | 0.0806 | **0.0462** |
| (C) PO OM 1009A | 0.0379 | 0.0120 | 0.0066 | 0.0056 | 0.0181 | 0.0146 | 0.0396 | 0.0463 | **0.0226** |
| (D) PO OM 1009B | 0.0409 | 0.0515 | 0.0414 | 0.0511 | 0.0484 | 0.0300 | 0.0270 | | **0.0415** |

Table 22: individual IL5 lung levels in mice.

| pg/mg | mouse 1 | mouse 2 | mouse 3 | mouse 4 | mouse 5 | mouse 6 | mouse 7 | mouse 8 | Mean |
|---|---|---|---|---|---|---|---|---|---|
| (A) PBS | 0 | 0.0004 | 0.0043 | 0 | | | | | **0.0012** |
| (B) LACK (asthmatic mice) | 0.1141 | 0.1002 | 0.1532 | 0.1115 | 0.3098 | 0.5173 | 0.5385 | 0.4547 | **0.2874** |
| (C) PO OM 1009A | 0.1817 | 0.0445 | 0.0572 | 0.0364 | 0.1643 | 0.0488 | 0.1917 | 0.1057 | **0.1038** |
| (D) PO OM 1009B | 0.1518 | 0.2798 | 0.1797 | 0.5439 | 0.3685 | 0.1320 | 0.1705 | | **0.2609** |

Table23: individual IL13 lung levels in mice.

| pg/mg | mouse 1 | mouse 2 | mouse 3 | mouse 4 | mouse 5 | mouse 6 | mouse 7 | mouse 8 | Mean |
|---|---|---|---|---|---|---|---|---|---|
| (A) PBS | 0 | 0 | 0 | 0.0208 | | | | | **0.0052** |
| (B) LACK (asthmatic mice) | 0.2727 | 0.0896 | 0.3542 | 0.2303 | 0.6178 | 1.1306 | 1.1768 | 1.0244 | **0.6120** |
| (C) PO OM 1009A | 0.3598 | 0.1231 | 0.0888 | 0.0995 | 0.2975 | 0.1909 | 0.5213 | 0.5463 | **0.2784** |
| (D) PO OM 1009B | 0.3628 | 0.8919 | 0.4291 | 0.7699 | 0.6441 | 0.4981 | 0.4972 | | **0.5847** |

The Th2 cytokines (IL4, IL5, and IL13) levels, when compared to the asthmatic control group (B), were decreased by the OM-1 009A extract, but not the OM-1009B extract. Hence, bacterial extracts obtained by a strong base treatment (OM-1009-B) may be less active than those obtained under a moderate base treatment (OM-1009-A). Even though both extracts were active in the Penh factor assay (Figure 6), OM-1009A may be a better candidate than OM-1009B for treatment or prevention of conditions related to Th2-related diseases, such as allergic diseases and atopy, or symptoms thereof.

**Claims**

1. An extract of one or more *Lactobacillus* bacterial strains, wherein the extract is soluble and wherein L-amino acids of proteins and lipopeptides of the *Lactobacillus* bacteria are at least partially racemized to D-amino acids.

2. The extract of claim 1, which comprises one or more of amino acid chosen among aspartic acid, glutamic acid, serine, histidine, alanine, arginine, tyrosine, methionine, phenylalanine, or lysine, racemized by at least 10%.

3. The extract of claim 1 or 2, which comprises an amount of nucleic acids less than 1 00µg/mL, or which comprises *Lactobacillus* bacterial membrane and cell wall molecules chosen among lipoproteins, lipopeptides, peptidoglycans, lipooligosaccharides, lipoteichoic acids, or teichoic acids cleaved in small structures by alkaline lysis and dissolved in said extract.

4. The extract of any one of the preceding claims, wherein the extract has immunomodulatory activity in a subject.

5. The extract of claim 4, wherein the extract has immunostimulatory activity in a subject.

6. The extract of claim 4, wherein the extract has anti-inflammatory activity in a subject.

7. The extract of any one of the preceding claims, wherein the one or more *Lactobacillus* strains comprise one or more of *Lactobacillus fermentum, Lactobacillus rhamnosus, Lactobacillus plantarum, Lactobacillus johnsonii, Lactobacillus helveticus, Lactobacillus casei defensis, Lactobacillus casei ssp. casei, Lactobacillus paracasei, Lactobacillus bulgaricus, Lactobacillus paracasei, Lactobacillus acidophilus, Lactobacillus reuteri, Lactobacillus salivarius, Lactobacillus lactis,* and *Lactobacillus delbrueckii.*

8. The extract of any one of the preceding claims, wherein the one or more *Lactobacillus* bacterial strains comprises one or more of *Lactobacillus fermentum I-*3929, *Lactobacillus rhamnosus* 71.38, *Lactobacillus plantarum* 71.39, *Lactobacillus johnsonii* 103782, and *Lactobacillus helveticus* 103146.

9. The extract of any one of the preceding claims, wherein the extract is capable of achieving a calculable IL10/IL12 ratio in human peripheral blood mononuclear cells, said ratio being equal to or greater than the IL10/IL12 ratio achieved by a live *Lactobacillus* strain from which the extract is obtained.

10. The extract of any one of the preceding claims, wherein the extract decreases the eosinophil cell number, neutrophil cell number, lymphocyte cell number, or any combination thereof, in an asthmatic murine subject by a factor of at least 1.5 with respect to an asthmatic non-treated control.

11. A nutraceutical composition or pharmaceutical composition comprising the extract of any one of the preceding claims.

12. The extract of any one of the preceding claims for use as a medicament.

13. The extract of any one of the preceding claims for use in a method of for treating and/or preventing and/or for reducing the symptoms in a subject of a condition selected in the group consisting of a respiratory disorder, an allergic condition, an urinary tract disorder, and a digestive disorder.

14. The extract of claim 13, wherein the respiratory disorder is chosen from pulmonary upper and lower infections, nasopharyngitis, sinusitis, pharyngitis, tonsilitis, laryngitis, tracheitis, laryngopharyngitis, influenza, pneumonia, bronchopneumonia, or obstructive pulmonary disease with acute exacerbation, wherein the allergic condition is chosen from allergic rhinitis, allergic asthma, or atopic dermatitis, wherein the urinary tract disorder is chosen from urethritis, tubulo-interstitial nephritis, obstructive pyelonephritis, cystitis including chronic cystitis, male pelvic pain syndrome including prostatitis and chronic prostatitis, prostatocystitis, or female pelvic inflammatory diseases, and wherein the digestive disorder is chosen from Crohn's disease or irritable bowel syndrome.

15. The extract of claim 13 and 14, wherein the subject is a human or a domestic animal.

# Figure 1

| TLRs | Ligands | Origin of ligands |
|---|---|---|
| TLR1/2 | Triacyl lipopeptides (Pam$_3$CSK$_4$) | Bacteria, mycobacteria |
| | Soluble factors | *Neisseria meningitides* |
| | OspA | *Borrelia burgdorferi* |
| | Porin PorB | *Neisseria meningitidis* |
| TLR2 | Lipoprotein/lipopeptides | A variety of pathogens |
| | Diacyl lipopeptides (Pam$_2$CSK$_4$ and MALP2SK$_4$) | Synthetic ligands |
| | Peptidoglycan | Gram-positive bacteria (not accessible in gram negative) |
| | Lipoteichoic acid | Gram-positive bacteria |
| | Lipoarabinomannan | Mycobacteria |
| | A phenol-soluble modulin | *Staphylococcus* epidermidis |
| | Glycoinositolphospholipids | *Trypanosoma cruzi* |
| | Glycolipids | *Treponema maltophilum* |
| | Porins | *Neisseria meningitidis* |
| | Zymosan | Fungi |
| | Atypical LPS | *Leptospira interrogans* |
| | Atypical LPS | *Porphyromonas gingivalis* |
| | Hsp70 | Host |
| | Hyaluronan | Host |
| | Hemagglutinin | Measles virus |
| TLR3 | Poly (I-C) dsRNA | Virus |
| TLR4 | LPS | Gram-negative bacteria |
| | Flavolipin | *Flavobacterium meningosepticum* |
| | ER-112022, E5564, E5531 | Synthetic compounds |
| | Taxol | Plant |
| | Fusion protein | Respiratory syncytial virus |
| | Envelope proteins | Mouse mammary tumor virus |
| | Hsp60 | *Chlamydia pneumoniae* |
| | Hsp60 | Host |
| | Hsp70 | Host |
| | Type III repeat extra domain A of fibronectin | Host |
| | Oligosaccharides of hyaluronic acid | Host |
| | Polysaccharide fragments of heparan sulfate | Host |
| | Fibrinogen | Host |
| | αA crystallin and HSPB8 | Host (recombinant *E. coli*–produced proteins) |
| TLR5 | Flagellin | Bacteria |
| TLR6/2 | Diacyl lipopeptides | Mycoplasma |
| TLR7 | Imidazoquinolines (imiquimod, R-848) | Synthetic compounds |
| | Bropirimine | Synthetic compounds |
| | Guanosine analogs | Synthetic compounds |
| TLR8 | R-848 | Synthetic compounds |
| TLR9 | Unmethylated CpG DNA | Bacteria, virus, yeast, insects |
| | Chromatin-IgG complexes | Host |

# Figure 2

# Figure 3/6

# Figure 4a

# Figure 4b

# Figure 5

## Figure 5a

## Figure 5b

# Figure 6

**EUROPEAN SEARCH REPORT**

Application Number

EP 12 18 3621

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 515 891 A (YOKOGAWA KANAE [JP] ET AL) 7 May 1985 (1985-05-07) <br> * column 5, line 4 - line 45; examples 1-4 * <br> * Reference examples 5-7 * | 1-7,9-12 | INV.<br>C12N1/20<br>A61K35/74<br>A61P11/00<br>A61P11/06<br>A61P37/08 |
| X | BILLOT-KLEIN DANIELLE ET AL: "Peptidoglycan structure of Lactobacillus casei, a species highly resistant to glycopeptide antibiotics", JOURNAL OF BACTERIOLOGY, vol. 179, no. 19, 1997, pages 6208-6212, XP002687051, ISSN: 0021-9193 <br> * abstract * <br> * table 1 * | 1-7,9,10 | A61P13/00<br>A61P1/00<br>A61P31/04<br>C12R1/25<br>C12N1/06<br>C12R1/225 |
| X | WALLINDER I B ET AL: "CELL WALL AND PEPTIDOGLYCAN FROM Lactobacillus fermenti.", JOURNAL OF BACTERIOLOGY MAR 1971 LNKD-PUBMED:5547995, vol. 105, no. 3, March 1971 (1971-03), pages 918-926, XP002687052, ISSN: 0021-9193 <br> * abstract * <br> * MATERIALS AND METHODS: Preparation of cell walls; Isolation of peptidoglycan * <br> * RESLUTS Amino acids and amino sugars; table 2 * <br> * page 923, right-hand column, last line - page 924, right-hand column, line 2 * | 1-7,9,10 | |

TECHNICAL FIELDS SEARCHED (IPC)

A61K
A61P
C12N
C12R

-/--

The present search report has been drawn up for all claims

| Place of search | Date of completion of the search | Examiner |
|---|---|---|
| Munich | 13 November 2012 | Böhmerova, Eva |

EPO FORM 1503 03.82 (P04C01)

Europäisches
Patentamt

European
Patent Office

Office européen
des brevets

# EUROPEAN SEARCH REPORT

**Application Number**

EP 12 18 3621

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HUNGERER K D ET AL: "Structure of the cell wall peptidoglycan of Lactobacillus casei RO94.", BIOCHEMISTRY SEP 1969 LNKD- PUBMED:5367451, vol. 8, no. 9, September 1969 (1969-09), pages 3567-3577, XP002687053, ISSN: 0006-2960 * abstract * * Materials and methods: Organsim and Isolation of Cell Walls * * figure 4; table II * | 1-7,9,10 | |
| Y | WO 01/91791 A2 (CT FOR THE IMPROVEMENT OF HUMA [US]) 6 December 2001 (2001-12-06) * page 3, line 5 - line 17 * * examples 1-3 * * CLAIMS * | 1-15 | |
| Y | US 7 183 108 B1 (CAYUELA CHANTAL [FR] ET AL) 27 February 2007 (2007-02-27) * column 2, line 60 - column 3, line 19; claims 1-9 * | 1-15 | TECHNICAL FIELDS SEARCHED (IPC) |
| Y | GAVRILIN M V ET AL: "Selecting optimum thermal acid hydrolysis conditions for obtaining lactobacillus hydrolysates", PHARMACEUTICAL CHEMISTRY JOURNAL, KLUWER ACADEMIC PUBLISHERS-CONSULTANTS BUREAU, NE, vol. 41, no. 2, 1 February 2007 (2007-02-01), pages 115-118, XP019532539, ISSN: 1573-9031 * the whole document * | 1-15 | |

-/--

The present search report has been drawn up for all claims

| Place of search | Date of completion of the search | Examiner |
|---|---|---|
| Munich | 13 November 2012 | Böhmerova, Eva |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

Europäisches
Patentamt

European
Patent Office

Office européen
des brevets

## EUROPEAN SEARCH REPORT

**Application Number**

EP 12 18 3621

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SAMOILOV V A ET AL: "Analysis of the Composition of a New Preparation Based on Lactobacillus Hydrolysates", PHARMACEUTICAL CHEMISTRY JOURNAL, KLUWER ACADEMIC PUBLISHERS-CONSULTANTS BUREAU, NE, vol. 39, no. 3, 1 March 2005 (2005-03-01), pages 164-165, XP019290460, ISSN: 1573-9031 * abstract * * page 164, left-hand column, last paragraph - right-hand column, paragraph 1 * | 1-15 | |
| Y | BRUCKNER H ET AL: "Chirality of amino acids of microorganisms used in food biotechnology", CHIRALITY 1993 US, vol. 5, no. 5, 1993, pages 385-392, XP009164451, ISSN: 0899-0042 * abstract * * MATERIALS AND METHODS: Fermentation of Bacteria; isolation of Amino Acids * * RESULTS; table 1 * | 1-15 | |
| A | US 2003/049231 A1 (BAUR MARKUS [DE] ET AL) 13 March 2003 (2003-03-13) * paragraph [0082]; example 1; table 1 * | 1-15 | |

**TECHNICAL FIELDS SEARCHED (IPC)**

-/--

The present search report has been drawn up for all claims

| Place of search | Date of completion of the search | Examiner |
|---|---|---|
| Munich | 13 November 2012 | Böhmerova, Eva |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

| | Europäisches Patentamt European Patent Office Office européen des brevets | **EUROPEAN SEARCH REPORT** | Application Number EP 12 18 3621 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HATCHER G E ET AL: "Augmentation of macrophase phagocytic activity by cell-free extracts of selected lactic acid-producing bacteria", JOURNAL OF DAIRY SCIENCE, AMERICAN DAIRY SCIENCE ASSOCIATION, SAVOY, IL, US, vol. 76, no. 9, 1 January 1993 (1993-01-01), pages 2485-2492, XP003007308, ISSN: 0022-0302 * page 2486, left-hand column, paragraph 2 * * abstract * | 1-15 | |
| A | SHOLEVA Z ET AL: "SCREENING OF ANTIMICROBIAL ACTIVITIES AMONG BULGARIAN LACTOBACILLI STRAINS", JOURNAL OF CULTURE COLLECTIONS, vol. 2, 1997, pages 15-20, XP002512226, * page 16, left-hand column, last paragraph - right-hand column, paragraph 1 * | 1-15 | TECHNICAL FIELDS SEARCHED (IPC) |

The present search report has been drawn up for all claims

| Place of search | Date of completion of the search | Examiner |
|---|---|---|
| Munich | 13 November 2012 | Böhmerova, Eva |

## ANNEX TO THE EUROPEAN SEARCH REPORT
## ON EUROPEAN PATENT APPLICATION NO.

EP 12 18 3621

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2012

| Patent document cited in search report | | Publication date | Patent family member(s) | | Publication date |
|---|---|---|---|---|---|
| US 4515891 | A | 07-05-1985 | DE | 3301997 A1 | 04-08-1983 |
| | | | FR | 2520379 A1 | 29-07-1983 |
| | | | JP | 1047153 B | 12-10-1989 |
| | | | JP | 1569635 C | 10-07-1990 |
| | | | JP | 58126797 A | 28-07-1983 |
| | | | US | 4515891 A | 07-05-1985 |
| WO 0191791 | A2 | 06-12-2001 | AU | 5125201 A | 11-12-2001 |
| | | | US | 6827940 B1 | 07-12-2004 |
| | | | WO | 0191791 A2 | 06-12-2001 |
| US 7183108 | B1 | 27-02-2007 | AR | 021302 A1 | 03-07-2002 |
| | | | AT | 249230 T | 15-09-2003 |
| | | | AU | 1276300 A | 05-06-2000 |
| | | | CA | 2351982 A1 | 25-05-2000 |
| | | | DE | 69911237 D1 | 16-10-2003 |
| | | | DE | 69911237 T2 | 17-06-2004 |
| | | | DK | 1131080 T3 | 12-01-2004 |
| | | | EP | 1131080 A2 | 12-09-2001 |
| | | | ES | 2207305 T3 | 16-05-2004 |
| | | | FR | 2785809 A1 | 19-05-2000 |
| | | | PT | 1131080 E | 27-02-2004 |
| | | | US | 7183108 B1 | 27-02-2007 |
| | | | WO | 0028943 A2 | 25-05-2000 |
| US 2003049231 | A1 | 13-03-2003 | AR | 030925 A1 | 03-09-2003 |
| | | | AT | 471152 T | 15-07-2010 |
| | | | AU | 3011801 A | 03-07-2001 |
| | | | BR | 0016709 A | 03-09-2002 |
| | | | CA | 2395419 A1 | 28-06-2001 |
| | | | CN | 1434716 A | 06-08-2003 |
| | | | CN | 101385745 A | 18-03-2009 |
| | | | DK | 1244463 T3 | 20-09-2010 |
| | | | EP | 1110555 A1 | 27-06-2001 |
| | | | EP | 1244463 A1 | 02-10-2002 |
| | | | ES | 2345761 T3 | 01-10-2010 |
| | | | JP | 4828759 B2 | 30-11-2011 |
| | | | JP | 2003518070 A | 03-06-2003 |
| | | | KR | 20020068382 A | 27-08-2002 |
| | | | MX | PA02006222 A | 05-12-2002 |
| | | | NO | 20022989 A | 16-08-2002 |
| | | | PL | 355411 A1 | 19-04-2004 |
| | | | PT | 1244463 E | 07-09-2010 |
| | | | US | 2003049231 A1 | 13-03-2003 |
| | | | US | 2006002910 A1 | 05-01-2006 |
| | | | WO | 0145721 A1 | 28-06-2001 |

EPO FORM P0459

For more details about this annex : see Official Journal of the European Patent Office, No. 12/82

**EP 2 540 818 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LAVELLE et al.** *Curr Top Med Chem.,* 2004, vol. 4 (5), 499-508
- **GAY ; GANGLOFF.** *Ann. Rev. Biochem.,* 2007, vol. 76, 141-65
- **TSE ; HORNER.** *Ann Rheum Dis.,* November 2007, vol. 66 (3), iii77-80
- **MOTTET et al.** *Digestive and Liver Disease,* 2005, vol. 37, 3-6
- **EZENDAM et al.** *Nutr Rev, Jan.,* 2006, vol. 64 (1), 1-14
- **GILL ; PRASAD.** *Adv Exp Med Biol,* 2008, vol. 606, 423-54
- **BHAKDI et al.** *Infect. Immun.,* 1991, vol. 59, 4614-4620
- **SETOYAMA et al.** *J Gen Microbiol,* 1985, vol. 131 (9), 2501-2503
- **CLEVELAND et al.** *Infect Immun,* 1996, vol. 64 (6), 1906-1912
- **DEININGER et al.** *Clin Vaccine Immunol,* 2007, vol. 14 (2), 1629-1633
- **T. AMBROUCHE.** Contribution à l'étude du pouvoir immunomodulateur des bifidobacteries: Analyse in vitro et étude ex vivo des mécanismes moléculaires impliqués. *Ph.D. Thesis,* 2005

- Lactobacillus Sepsis Associated With Probiotic Therapy. *Pediatrics,* January 2005, vol. 115 (1), 178-181
- **DE VRESE et al.** *J Nutrition,* 2000, 2026-2031
- Separations Technology, Pharmaceutical and Biotechnology Applications. Interpharm Press, Inc, 126-135
- **M.CHERYAN.** Ultrafiltration and Microfiltration Handbook. 1998
- **FOLIGNE et al.** *World J Gastroenterol,* 2007, vol. 13 (2), 236-243
- **HERBERT et al.** *Meth. Microbiol,* 1971, vol. 5B, 266
- **FOLIGNE et al.** *World J Gastroenterol,* 14 January 2007, vol. 13 (2), 236-243
- **BURDELYA et al.** An agonist of toll-like receptor 5 has radioprotective activity in mouse and primate models. *Science,* 2008, vol. 320, 226-30
- **CUNNINGHAM ; SEENBERG.** *Immunology,* 1968, vol. 14, 599
- **I. MECHNIKOV.** *Institute for Vaccines and Sera, Russian Academy of Medical Sciences*
- **JULIA et al.** *Immunity,* 2002, vol. 16, 271-283